# FASCICULE DE BREVET EUROPEEN

(11) **EP 0 728 739 B1**
(45) Date de publication et mention de la délivrance du brevet: **05.11.1997**
(21) Numéro de dépôt: 96400251.3
(22) Date de dépôt: 06.02.1996
(51) Int. Cl.: C07C 251/54, C07C 251/48, C07C 251/56, C07C 251/58, C07C 233/00, C07C 235/00, C07C 237/00, A61K 31/15, A61K 7/48, A61K 7/06

(54) **Composés bi-aromatiques dérivés d'amide, compositions pharmaceutiques et cosmétiques les contenant et utilisations**
Biaromatische Amidderivate, diese enthaltende pharmazeutische und kosmetische Zusammensetzungen und ihre Verwendung
Biaromatic amide derivatives, pharmaceutical and cosmetic compositions containing them and their utilisations

(30) Priorité: 23.02.1995 FR 9502133
(43) Date de publication de la demande: 28.08.1996
(73) Titulaire: CENTRE INTERNATIONAL DE RECHERCHES DERMATOLOGIQUES GALDERMA (C.I.R.D. GALDERMA), F-06560 Valbonne (FR)
(72) Inventeur: Bernardon, Jean-Michel, F-06650 Le Rouret (FR); Vigne, Laurence, Résidence Paradis Parc, Bât A, F-06600 Antibes (FR)
(74) Mandataire: Tezier Herman, Béatrice

(56) Documents cités:
- EP-A- 0 227 431
- EP-A- 0 514 264
- EP-A- 0 661 260
- WO-A-92/06948

## Description

L'invention concerne, à titre de produits industriels nouveaux et utiles, des composés bi-aromatiques dérivés d'amide. Elle concerne également l'utilisation de ces nouveaux composés dans des compositions pharmaceutiques destinées à un usage en médecine humaine ou vétérinaire, ou bien encore dans des compositions cosmétiques.

Il a déjà été décrit dans les demandes de brevets WO 92/06948 et EP 0 661 260, déposées par la Demanderesse, d'autres composés biaromatiques dérivés d'amide.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies. Les composés selon l'invention peuvent par ailleurs trouver une application intéressante dans le traitement de I'ostéoporose, ou dans le traitement des maladies virales, ainsi que dans le traitement de tout état associé à une hypervitaminose A. D'une manière générale, ils peuvent enfin trouver une application dans le traitement de toute maladie qui est associée à une modification de l'expression des récepteurs appartenant à la superfamille des récepteurs des hormones stéroïdiennes et thyroïdiennes.

On peut également utiliser les composés selon l'invention dans des compositions cosmétiques pour l'hygiène corporelle et capillaire.

Les composés selon l'invention peuvent être représentés par la formule générale (I) suivante : 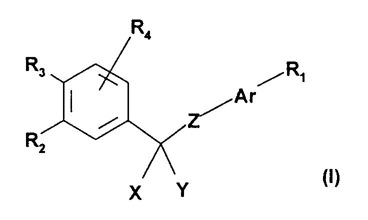 dans laquelle :
∗ Ar représente un radical choisi parmi les radicaux de formules (a)-(e) suivantes: 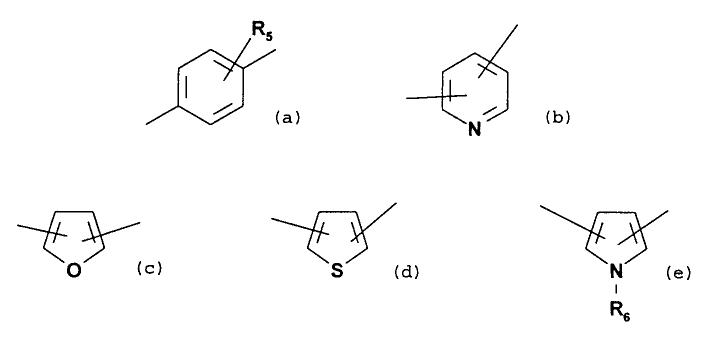
   R₅ et R₆ ayant les significations données ci-après,
∗ Z représente -CO-NR₇-,
∗ R₁ représente :
   (i) un radical -CH₃
   (ii) un radical -CH₂-O-R₆
   (iii) un radical -O-R₆
   (iv) un radical -CO-R₈,
   (v) un radical -S(O)ₜR₉

   R₆, R₈, R₉ et t ayant les significations données ci-après,
* soit X et Y peuvent être pris séparement :
   X représente un atome d'hydrogène ou un radical alkyle inférieur de C₁ à C₆,
   Y représente un radical de formule -(CH₂)ₙ-CH₃ ou de formule -(CH₂)ₙ-R₁₀
* soit X et Y peuvent être pris ensemble :
   X et Y forment un radical unique à double liaison choisi parmi les radicaux de formules :
      - =N-C-(CH₂)ₙ-R₁₀
      - =CH-(CH₂)ₘ-R₁₀
      - =N-O-(CH₂)₂-O-CH₂-CH₃
      - =N-O-CH₂-O-CH₂-CH₂-O-CH₃
      - =CH-(CH₂)ₘ-CH₃
   R₁₀, m et n ayant les significations données ci-après,
∗ R₂ et R₃, pris séparément, représentent un atome d'hydrogène, un radical alkyle linéaire ou ramifié ayant de 1 à 20 atomes de carbone, un radical -OR₆ ou un radical -SR₆, R₆ ayant la signification donnée ci-après, étant entendu que R₂ et R₃ ne peuvent pas être simultanément un atome d'hydrogène, un radical -OR₆ ou un radical -SR₆, ou R₂ et R₃, pris ensemble, forment avec le cycle aromatique adjacent un cycle à 5 ou 6 chainons éventuellement substitué par des groupes méthyle et/ou éventuellement interrompu par un atome d'oxygène ou de soufre ou par un radical -SO- ou SO₂-,
∗ R₄ représente un atome d'hydrogène, un atome d'halogène, un radical alkyle inférieur de C₁ à C₆ ou un radical -OR₆, R₆ ayant la signification donnée ci-après,
étant entendu que dans tout ce qui précède :
- R₅ a la même signification que R₄,
- R₆ représente un atome d'hydrogène, un radical alkyle linéaire ou ramifié ayant de 1 à 20 atomes de carbone ou un radical -CO-R₁₁, R₁₁ ayant la signification donnée ci-après,
- R₇ représente un atome d'hydrogène ou un radical alkyle inférieur de C₁ à C₆,
- R₈ représente :
   (a) un atome d'hydrogène
   (b) un radical alkyle inférieur de C₁ à C₆
   (c) un radical de formule : 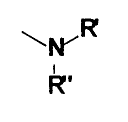 R' et R'' ayant les significations données ci-après,
   (d) un radical -OR₁₂, R₁₂ ayant la signification donnée ci-après,
- R₉ et R₁₁, identiques ou différents, représentent un radical alkyle inférieur de C₁ à C₆,
- R₁₀ représente :
   (i) un radical -OR₆
   (ii) un radical -CO-R₈
   (iii) un radical de formule 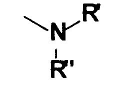 R' et R'' ayant les significations données ci-dessous,
   (iv) un radical aryle, éventuellement substitué,
   (v) un radical hétérocycle,
   (vi) un radical cycloaliphatique en C₃ ou C₆,
- R₁₂ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,
- R' et R'', identiques ou différents, représentent un atome d'hydrogène, un radical alkyle inférieur de C₁ à C₆, un radical mono ou polyhydroxyalkyle, un radical aryle éventuellement substitué ou un reste d'aminoacide ou de peptide ou de sucre ou encore, pris ensemble, forment un hétérocycle,
- m est un nombre entier de 1 à 20,
- n est un nombre entier de 2 à 20,
- t est un nombre entier égal à 0,1 ou 2,

L'invention vise également les sels des composés de formule (I) ci-dessus dans le cas où R₁ représente une fonction acide carboxylique, ainsi que les isomères optiques et géométriques desdits composés. Lorsque les composés selon l'invention se présentent sous forme de sels, il s'agit de préférence de sels d'un métal alcalin ou alcalino-terreux, ou encore de zinc ou d'une amine organique.

Selon la présente invention, parmi les radicaux alkyle inférieur ayant de 1 à 6 atomes de carbone, on préfère les radicaux méthyle, éthyle, isopropyle, butyle, tertiobutyle et hexyle.

Par radical alkyle linéaire ou ramifié ayant de 1 à 20 atomes de carbone, on entend notamment les radicaux méthyle, éthyle, propyle, 2-éthyl-hexyle, octyle, dodécyle, hexadécyle et octadécyl.

Par radical monohydroxyalkyle, on entend un radical ayant de préférence 2 ou 3 atomes de carbone, notamment un radical 2-hydroxyéthyle, 2-hydroxypropyle ou 3-hydroxypropyle.

Par radical polyhydroxyalkyle, on entend un radical contenant de préférence de 3 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical aryle, on entend de préférence un radical phényle éventuellement substitué par au moins un atome d'halogène, un hydroxyle ou une fonction nitro.

Par radical aralkyle, on entend de préférence le radical benzyle ou phénéthyle éventuellement substitué par au moins un atome d'halogène, un hydroxyle ou une fonction nitro.

Par radical alkényle, on entend un radical contenant de préférence de 1 à 5 atomes de carbone et présentant une ou plusieurs insaturations éthyléniques, tel que plus particulièrement le radical allyle.

Par reste de sucre, on entend un reste dérivant notamment de glucose, de galactose ou de mannose, ou bien encore de l'acide glucuronique.

Par reste d'aminoacide, on entend notamment un reste dérivant de la lysine, de la glycine ou de l'acide aspartique, et par reste de peptide on entend plus particulièrement un reste de dipeptide ou de tripeptide résultant de la combinaison d'acides aminés.

Par radical cycloaliphatique en C3 ou C6, on peut citer plus particulèrement le radical cyclopropyle ou cyclohexyle.

Par hétérocycle enfin, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Lorsque R₄ et R₅ représente un atome d'halogène, celui-ci est de préférence un atome de fluore, de chlore et de brome.

Parmi les composés de formule (I) ci-dessus rentrant dans le cadre de la présente invention, on peut notamment citer les suivants :
- Acide *anti*-4-[α-hydroxyhexylimino-(5,6,7,8-tétrahydro-5,5,8,8-tétraméthyl-2-naphtyl)acétamido]benzoïque
- Acide *syn*-4-[α-hydroxyhexylimino-(5,6,7,8-tétrahydro-5,5,8,8-tétraméthyl-2-naphtyl)acétamido]benzoïque
- Acide *syn*-4-[α-méthoxycarbonylpentylimino-(5,6,7,8-tétrahydro-5,5,8,8-tétraméthyl-2-naphtyl)acétamido]benzoïque
- Acide syn-4-[α-hydroxyoctyloxyimino-(5,6,7,8-tétrahydro-5,5,8,8-tétraméthyl-2-naphtyl)acétamido]benzoïque.
- Acide trans-4-[α-hydroxyoctyloxyimino-(5,6,7,8-tétrahydro-5,5,8,8-tétraméthyl-2-naphtyl)acétamido]benzoïque.
- Acide syn-4-[α-hydroxyheptyloxyimino-(5,6,7,8-tétrahydro-5,5,8,8-tétraméthyl-2-naphtyl)acétamido]benzoïque.
- Acide trans-4-[α-hydroxyheptyloxyimino-(5,6,7,8-tétrahydro-5,5,8,8-tétraméthyl-2-naphtyl)acétamido]benzoïque.
- Acide syn-4-[α-hydroxypropyloxyimino-(5,6,7,8-tétrahydro-5,5,8,8-tétraméthyl-2-naphtyl)acétamido]benzoïque.
- Acide trans-4-[α-hydroxypropyloxyimino-(5,6,7,8-tétrahydro-5,5,8,8-tétraméthyl-2-naphtyl)acétamido]benzoïque.
- Acide syn-4-[α-éthoxycarbonylpropyloxyimino-(5,6,7,8-tétrahydro-5,5,8,8-tétraméthyl-2-naphtyl)acétamido]benzoïque.
- Acide syn-4-[α-hydroxydecyloxyimino-(5,6,7,8-tétrahydro-5,5,8,8-tétraméthyl-2-naphtyl)acétamido]benzoïque.
- Acide syn-4-[α-hydroxynonyloxyimino-(5,6,7,8-tétrahydro-5,5,8,8-tétraméthyl-2-naphtyl)acétamido]benzoïque.
- Acide syn-4-[α-benzyloxyimino-(5,6,7,8-tétrahydro-5,5,8,8-tétraméthyl-2-naphtyl)acétamido]benzoïque.
- Acide syn-4-[α-methoxyethoxymethoxyimino-(5,6,7,8-tétrahydro-5,5,8,8-tétraméthyl-2-naphtyl)acétamido]benzoïque.
- syn-4-[α-éthoxycarbonylpropyloxyimino-(5,6,7,8-tétrahydro-5,5,8,8-tétraméthyl-2-naphtyl)acétamido]benzoate d'allyle.
- Acide (E)-4-[2-(5,6,7,8-tétrahydro-5,5,8,8-tétraméthyl-2-naphtyl)-2-nonenamido]benzoïque.
- Acide (Z)-4-[2-(5,6,7,8-tétrahydro-5,5,8,8-tétraméthyl-2-naphtyl)-2-nonenamido]benzoïque.
- Acide (E)-4-[2-(5,6,7,8-tétrahydro-5,5,8,8-tétraméthyl-2-naphtyl)-2-undecenamido]benzoïque.
- (E)-4-[2-(5,6,7,8-tétrahydro-5,5,8,8-tétraméthyl-2-naphtyl)-2-undecenamido]phénylcarboxamide.
- (E)-4-[2-(5,6,7,8-tétrahydro-5,5,8,8-tétraméthyl-2-naphtyl)-2-undecenamido]benzèneméthanol.
- (E)-4-[2-(5,6,7,8-tétrahydro-5,5,8,8-tétraméthyl-2-naphtyl)-2-undecenamido]phénol.
- Acide (Z)-4-[2-(5,6,7,8-tétrahydro-5,5,8,8-tétraméthyl-2-naphtyl)-2-undecenamido]benzoïque.
- Acide(E)-4-[2-(5,6,7,8-tétrahydro-5,5,8,8-tétraméthyl-2-naphtyl)-2-hexadecenamido]benzoïque.
- Acide (Z)-4-[2-(5,6,7,8-tétrahydro-5,5,8,8-tétraméthyl-2-naphtyl)-2-hexadecenamido]benzoïque.

Selon la présente invention, les composés de formule (I) plus particulièrement préférés sont ceux pour lesquels R₁ représente le radical -COR₈, X et Y pris ensemble forment un radical choisi parmi les radicaux de formules suivantes :
- =N-O-(CH2)n-R₁₀
- =CH-(CH2)m-R₁₀
- =N-O-(CH₂)₂-O-CH₂-CH₃
- =N-O-CH₂-O-CH₂-CH₂-O-CH₃
- =CH-(CH₂)ₘ-CH₃,
R₈, R₁₀, m et n ayant les significations données ci-avant.

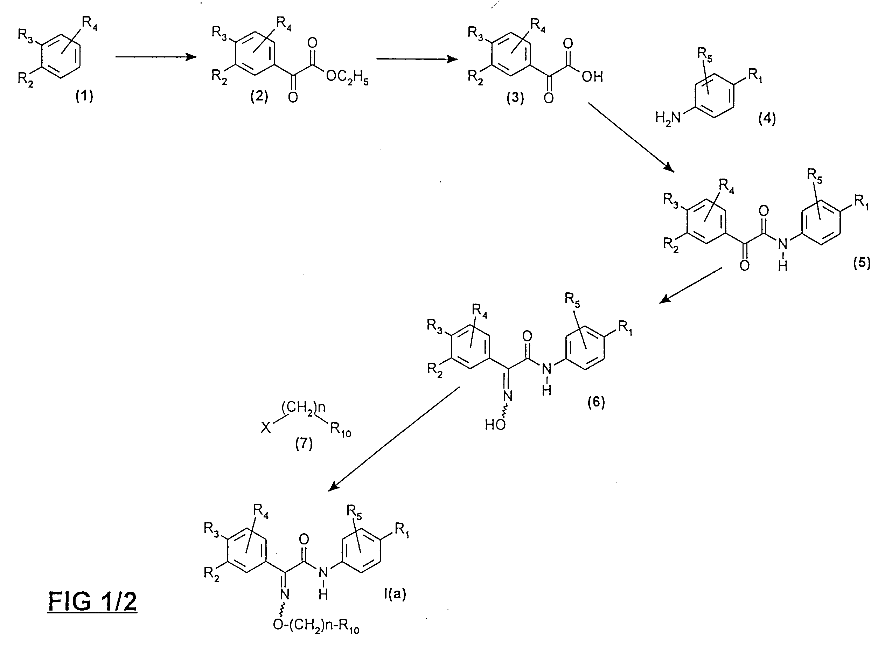

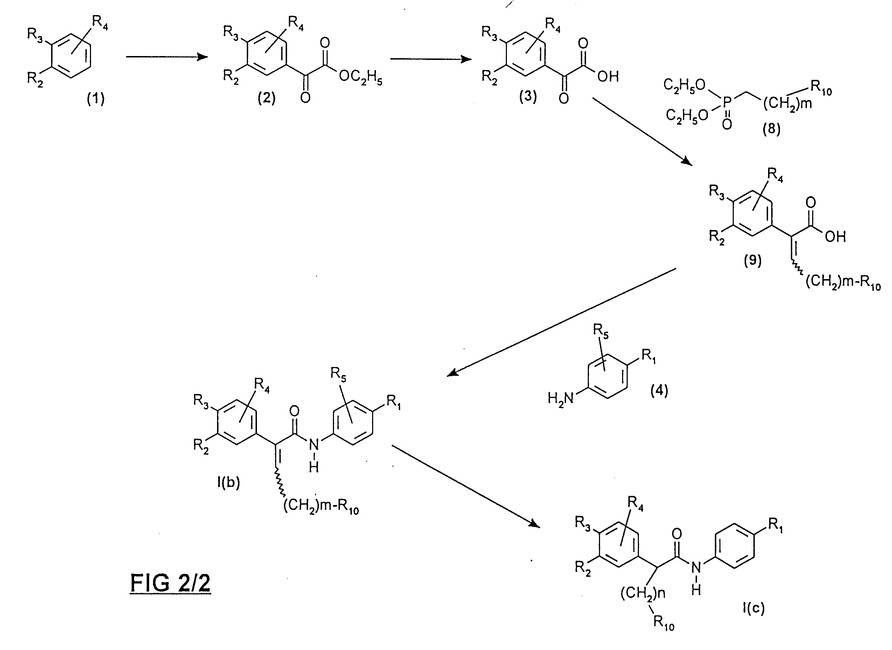

La présente invention a également pour objet les procédés de préparation des composés de formule (I) ci-dessus selon les schémas réactionnels donnés aux Figures 1 et 2.

Les dérivés de formule (Ia) peuvent être préparés par une suite de réactions **Figure 1** comprenant tout d'abord une réaction de type Friedel et Crafts entre un dérivé aromatique de formule (1) et le chlorure d'éthoxalyle en présence d'un acide de Lewis tel AlCl₃ puis saponification de la fonction ester en présence d'une base , telle l'hydroxyde de sodium ou de lithium dans un solvant alcoolique ou le THF.Le dérivé de formule (3) ainsi obtenu est couplé avec l'aniline de formule (4) soit par l'intermédiaire du chlorure d'acide ou en présence de dicyclohexylamine et de diméthylaminopyridine dans un solvant tel le THF ou le dichlorométhane.Par réaction du composé de formule (5) avec l'hydroxylamine dans un solvant alcoolique tel l'alcool éthylique on obtient le composé de formule (6) qui est ensuite alkylé par un dérivé halogéné de formule (7) de préférence iodé ou bromé soit par l'intermédiaire de son dérivé sodé dans le DMF, soit en présence de carbonate de potassium dans un solvant tel la méthyléthylcétone.

Les dérivés de formule (Ib) peuvent être préparés par une suite de réactions **Figure 2** mettant en jeu une réaction de type Horner-Emmons entre le dérivé glyoxylique de formule (3) et un phosphonate de formule (8). Le dérivé de formule (9) ainsi obtenu est ensuite couplé avec l'aniline de formule (4) soit par l'intermédiaire du chlorure d'acide ou en présence de dicyclohexylamine et de diméthylaminopyridine dans un solvant tel le THF ou le dichlorométhane.

Les dérivés de formule I(c) peuvent être obtenus à partir du dérivé de formule I(b) par hydrogénation avec un catalyseur tel le Palladium sur charbon dans un solvant tel le dioxanne ou l'alcool éthylique.

Lorsque R₁ représente le radical -COOH, les composés sont préférentiellement préparés en protégeant R₁ par un groupe protecteur de type allylique, benzylique ou tert-butylique.Le passage à la forme libre peut être éffectué:
- dans le cas d'un groupe protecteur allylique, au moyen d'un catalyseur tel que certains complexes de métaux de transition par exemple le tétrakis(triphénylphosphine)Palladium(0) en présence d'une amine secondaire telle la morpholine ou du sel de sodium du malonate de diéthyle.
- dans le cas d'un groupe protecteur benzylique, par débenzylation en présence d'hydrogène, au moyen d'un catalyseur tel le palladium sur charbon dans un solvant alcoolique tel l'alcool éthylique ou dans le dioxanne.
- dans le cas d'un groupe protecteur tert-butylique au moyen d'iodure de triméthylsilane ou d'acide trifluoroacétique dans un solvant tel que le dichlorométhane.

La présente invention a également pour objet à titre de médicament les composés de formule (I) telle que définie ci-dessus.

Selon la nature des radicaux retenus, ces composés pourront présenter soit une activité agoniste dans le test de différenciation des cellules (F9) de tératocarcinome embryonnaire de souris (Cancer Research 43, p. 5268, 1983) et/ou dans le test d'inhibition de l'ornithine décarboxylase après induction par le TPA chez la souris (Cancer Research 38, p. 793-801, 1978), soit au contraire, une activité antagoniste vis-à-vis de l'expression d'un ou plusieurs marqueurs biologiques dans le test de différenciation des cellules (F9) de tératocarcinome embryonnaire de souris (Skin pharmacol., 3, pp 156-267, 1990) et/ou sur la différenciation des kératinocytes humains in vitri (Anal. Biochem., 192, pp 232-236, 1991).

Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnées vulgaires, comédoniennes, polymorphes, rosacées, les acnées nodulokystiques, conglobata, les acnées séniles, les acnées secondaires telles que l'acnée solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation ou pour prévenir ou pour réparer les vergetures,
10) pour lutter contre les troubles de la fonction sébacée tels que l'hyperséborrhée de l'acnée ou la séborrhée simple,
11) dans le traitement ou la prévention des états cancéreux ou précancéreux,
12) dans le traitement d'affections inflammatoires telles que l'arthrite,
13) dans le traitement de toute affection d'origine virale au niveau cutané ou général,
14) dans la prévention ou le traitement de l'alopécie,
15) dans le traitement d'affections dermatologiques ou générales à composante immunologique,
16) dans le traitement d'affections du système cardiovasculaire telles que l'artériosclérose,
17) dans le traitement ou la prévention de l'ostéoporose,
18) dans le traitement des dépressions saisonnières.

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutate, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses isomères optiques ou géométriques ou un de ses sels.

La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses isomères optiques ou géométriques ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crêmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001% et 5% en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques, tous ces différents produits étant tels que définis ci-avant.

La présente invention vise donc également une composition cosmétique qui est caractérisée par le fait qu'elle comprend, dans un support cosmétiquement acceptable et convenant à une application topique, au moins un composé de formule (I) telle que définie ci-dessus ou l'un de ses isomères optiques ou géométriques ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques selon l'invention est avantageusement comprise entre 0,001% et 3% en poids par rapport à l'ensemble de la composition.

Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétokonazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,4-diphénylimidazolidine 2,4-dione); des agents anti-inflammatoires non stéroïdiens; des caroténoïdes et, notamment, le β-carotène; des agents anti-psoriatiques tels que l'anthraline et ses dérivés; et enfin les acides eicosa-5,8,11,14-tétraynoïque et eicosa-5,8,11-trynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

On va maintenant donner, à titre d'illustration et sans aucun caractère limitatif, plusieurs exemples d'obtention de composés actifs de formule (I) selon l'invention, ainsi que diverses formulations concrètes à base de tels composés.

### EXEMPLE 1

### syn-4-[α-hydroxyhexyloxyimino-(5,6,7,8-tétrahydro-5,5,8,8-tétraméthyl-2-naphtyl)acétamido]benzoate d'allyle.

### (a) 5,6,7,8-tétrahydro-5,5,8,8-tétraméthyl-2-naphtylglyoxylate d'éthyle.

Dans un tricol, on introduit 14,5 g (0,109 mole) de chlorure d'aluminium, 100 ml de dichlorométhane et ajoute goutte à goutte un mélange de 11,7 g (62 mmoles) de 5,6,7,8-tétrahydro-5,5,8,8-tétraméthyl-2-naphtalène et de 8 ml (71 mmoles) de chlorure d'étoxalyle dans 100 ml de dichlorométhane et agite à température ambiante pendant une heure. On verse le milieu réactionnel dans la glace, extrait avec de l'éther éthylique, décante la phase organique, sèche sur sulfate de magnésium, évapore. Le résidu obtenu est purifié par chromatographie sur colonne de silice élué avec un mélange de dichlorométhane et d'hexane (30-70). Après évaporation des solvants, on recueille 16,5 g (93%) de l'ester attendu sous forme d'une huile légèrement jaune.

### (b) Acide 5,6,7,8-tétrahydro-5,5,8,8-tétraméthyl-2-naphtylglyoxylique.

Dans un ballon, on introduit 16 g (55,6 mmoles) de 5,6,7,8-tétrahydro-5,5,8,8-tétraméthyl-2-naphtylglyoxylate d'éthyle et 50 ml d'alcool éthylique. On ajoute une solution de 2,5 g (58,5 mmoles) d'hydroxyde de sodium dans 50 ml d'eau et chauffe à reflux pendant une heure. On évapore à sec le milieu réactionnel, reprend par l'eau et l'éther éthylique, décante la phase aqueuse, acidifie à pH 1 avec de l'acide chlorhydrique concentré, extrait avec de l'éther éthylique, décante la phase organique, sèche sur sulfate de magnésium, évapore. On recueille 14 g (97%) d'acide 5,6,7,8-tétrahydro-5,5,8,8-tétraméthyl-2-naphtylglyoxylique, sous forme d'une huile incolore.

### (c) Chlorure de 5,6,7,8-tétrahydro-5,5,8,8-tétraméthyl-2-naphtylglyoxyloyle.

Dans un ballon, on introduit 2,6 g (10 mmoles) d'acide 5,6,7,8-tétrahydro-5,5,8,8-tétraméthyl-2-naphtylglyoxylique dans 50 ml de dichlorométhane, ajoute 2 ml (10 mmoles) de dicyclohexylamine et agite à température ambiante pendant une heure. On ajoute ensuite goutte à goutte 800 µl (10 mmoles) de chlorure de thionyle et agite à température ambiante pendant une heure. On évapore à sec, reprend par l'éther éthylique, filtre le sel de dicyclohexylamine et évapore. On obtient 2,8 g (100%) de chlorure d'acide brut, qui est utilisé tel quel pour la suite de la synthèse.

### (d) 4-(5,6,7,8-tétrahydro-5,5,8,8-tétraméthyl-2-naphtoylcarboxamido)benzoate d'allyle.

Dans un ballon, on introduit 3,6 g (20 mmoles) de 4-aminobenzoate d'allyle 2,8 ml (20 mmoles) de triéthylamine et 50 ml de THF. On ajoute goutte à goutte une solution de 5,6 g (20 mmoles) de chlorure de 5,6,7,8-tétrahydro-5,5,8,8-tétraméthyl-2-naphtylglyoxyloyle dans 50 ml de THF et agite à température ambiante pendant quatre heures. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec un mélange de dichlorométhane et d'hexane (40-60). Après évaporation des solvants, on recueille 2 g (46%) de l'ester allylique attendu sous forme d'une huile légèrement jaune.

### (e) 4-[α-hydroxyimino-(5,6,7,8-tétrahydro-5,5,8,8-tétraméthyl-2-naphtyl)acétamido]benzoate d'allyle.

Dans un ballon, on introduit 1,7 g (4 mmoles) de 4-(5,6,7,8-tétrahydro-5,5,8,8-tétraméthyl-2-naphtoylcarboxamido)benzoate d'allyle 1,1 g (16 mmoles) de chlorhydrate d'hydroxylamine et 50 ml d'éthanol. On ajoute goutte à goutte 16 ml de NaOH (1N) et chauffe à reflux pendant 4 heures. On évapore le milieu réactionnel à sec, reprend le résidu dans l'eau, extrait avec de l'éther éthylique, décante la phase organique, sèche sur sulfate de magnésium, évapore. Le résidu obtenu est chromatographié sur colonne de silice, élué avec un mélange de dichlorométhane et d'éther éthylique (98-2). Après évaporation des solvants, on obtient 1,6 g (94%) du produit attendu de point de fusion 176-7°C.

### (f) syn-4-[α-hydroxyhexyloxyimino-(5,6,7,8-tétrahydro-5,5,8,8-tétraméthyl-2-naphtyl)acétamido]benzoate d'allyle.

Dans un ballon, on introduit 3,25 g (7,5 mmoles) de 4-[α-hydroxyimino-(5,6,7,8-tétrahydro-5,5,8,8-tétraméthyl-2-naphtyl)acétamido]benzoate d'allyle 1 g (7,5 mmoles) de carbonate de potassium et 100 ml de méthyléthylcétone. On ajoute 1 ml (7,5 mmoles) de 6-bromohexanol et chauffe à reflux pendant 24 heures. On filtre le milieu réactionnel, évapore à sec le filtrat, reprend par l'eau et l'éther éthylique, décante la phase organique, sèche sur sulfate de magnésium, évapore. Le résidu obtenu est purifié par chromatographie sur colonne de silice élué avec du dichlorométhane. On recueille ainsi 3,3 g (82%) du produit attendu sous forme d'une huile jaune.

### EXEMPLE 2

### Acide syn-4-[α-hydroxyhexyloxyimino-(5,6,7,8-tétrahydro-5,5,8,8-tétraméthyl-2-naphtyl)acétamido]benzoïque.

Dans un tricol et sous courant d'azote, on introduit 3,3 g (6,2 mmoles)de *syn*-4-[α-hydroxyhexyloxyimino-(5,6,7,8-tétrahydro-5,5,8,8-tétraméthyl-2-naphtyl) acétamido]benzoate d'allyle, 230 mg (0,2 mmole) de tétrakis(triphénylphosphine) palladium(0), et 100 ml de THF. On ajoute ensuite goutte à goutte 2,7 ml (31 mmoles) de morpholine et agite à température ambiante pendant 2 heures. On évapore le milieu réactionnel à sec, reprend par l'eau, acidifie à pH 1, extrait avec de l'éther éthylique, décante la phase organique, lave à l'eau, sèche sur sulfate de magnésium, évapore. Le résidu obtenu est purifié par chromatographie sur colonne de silice élué avec de l'éther éthylique, on recueille 2 g (65,5%) d'acide *syn*-4-[α-hydroxyhexyloxyimino-(5,6,7,8-tétrahydro-5,5,8,8-tétraméthyl-2-naphtyl)acétamido]benzoïque de point de fusion 183-6°C.

### EXEMPLE 3

### Acide anti-4-[α-hydroxyhexyloxyimino-(5,6,7,8-tétrahydro-5,5,8,8-tétraméthyl-2-naphtyl)acétamido]benzoïque.

Dans un réacteur photochimique, on introduit 800 mg (1,7 mmoles) d'acide *syn*-4-[α-hydroxyhexyloxyimino-(5,6,7,8-tétrahydro-5,8,8,8-tétraméthyl-2-naphtyl)acétamido]benzoïque, 600 ml de méthanol et agite 48 heures à température ambiante en irradiant (lampe Hanovia moyenne pression, sans filtre). On évapore le milieu réactionnel, et purifie le résidu obtenu par chromatographie sur colonne de silice élué avec un mélange de dichlorométhane et de méthanol (98-2). Après évaporation des solvants, on recueille 470 mg (59%) d'acide *anti* 4-[α-hydroxyhexyloxyimino-(5,6,7,8-tétrahydro-5,5,8,8-tétraméthyl-2-naphtyl)acétamido] benzoïque de point de fusion 178-81°C.

### EXEMPLE 4

### syn-4-[α-éthoxycarbonylpentylxyimino-(5,6,7,8-tétrahydro-5,5,8,8-tétraméthyl-2-naphtyl)acétamido]benzoate d'allyle.

De manière analogue à l'exemple 1(f) par réaction de 3,25 g (7,5 mmoles) de 4-[α-hydroxyimino-(5,6,7,8-tétrahydro-5,5,8,8-tétraméthyl-2-naphtyl)acétamido]benzoate d'allyle avec 1,3 ml (7,5 mmoles) de 6-bromohexanoate d'éthyle, on obtient 1,7 g (39,5%) de *syn*-4-[α-éthoxycarbonylpentyloxyimino-(5,6,7,8-tétrahydro-5,5,8,8-tétraméthyl-2-naphtyl)acétamido]benzoate d'allyle sous forme d'une huile légèrement jaune.

### EXEMPLE 5

### Acide syn-4-[α-éthoxycarbonylpentyloxyimino-(5,6,7,8-tétrahydro-5,5,8,8-tétraméthyl-2-naphtyl)acétamido]benzoïque.

De manière analogue à l'exemple 2 à partir de 1,7 g (3 mmoles) de *syn*-4-[α-méthoxycarbonylpentyloxyimino-(5,6,7,8-tétrahydro-5,5,8,8-tétraméthyl-2-naphtyl)acétamido]benzoate d'allyle, on obtient 1,1 g (70%) d'acide syn-4-[α-éthoxycarbonylpentyloxyimino-(5,6,7,8-tétrahydro-5,5,8,8-tétraméthyl-2-naphtyl)acétamido]benzoïque de point de fusion 152-4°C.

### EXEMPLE 6

### Acide syn-4-[α-hydroxyoctyloxyimino-(5,6,7,8-tétrahydro-5,5,8,8-tétraméthyl-2-naphtylacétamio]benzoïque.

### (a) syn-4-[α-hydroxyoctyloxyimino-(5,6,7,8-tétrahydro-5,5,8,8-tétraméthyl-2-naphtyl)acétamido]benzoate d'allyle.

De manière analogue à l'exemple 1(f) par réaction de 2,2 g (5 mmoles) de 4-[α-hydroxyimino-(5,6,7,8-tétrahydro-5,5,8,8-tétraméthyl-2-naphtyl)acétamido] benzoate d'allyle avec 870 µl (5 mmoles) de 8-bromo-1-octanol, on recueille après purification par chromatographie sur colonne de silice élué avec du dichlorométhane 2 g (70%) de l'ester allylique attendu sous forme d'une huile incolore.

### (b) acide syn-4-[α-hydroxyoctyloxyimino-(5,6,7,8-tétrahydro-5,5,8,8-tétraméthyl-2-naphtyl)acétamido]benzoïque.

De manière analogue à l'exemple 2, à partir de 2 g (3,5 mmoles) de syn-4-[α-hydroxyoctyloxyimino-(5,6,7,8-tétrahydro-5,5,8,8-tétraméthyl-2-naphtyl)acétamido] benzoate d'allyle on obtient 1,3 g (70%) de l'acide attendu de point de fusion 102-4°C.

### EXEMPLE 7

### Acide, trans-4-[α-hydroxyoctyloxyimino-(5,6,7,8-tétrahydro-5,5,8,8-tétraméthyl-2-naphtyl)acétamido]benzoïque.

De manière analogue à l'exemple 3 à partir de 900 mg (1,7 mmole) d'acide syn-4-[α-hydroxyoctyloxyimino-(5,6,7,8-tétrahydro-5,5,8,8-tétraméthyl-2-naphtyl) acétamido]benzoïque, on obtient 390 mg (43%) de l'acide trans attendu de point de fusion 98-100°C.

### EXEMPLE 8

### Acide syn-4-[α-hydroxyheptyloxyimino-(5,6,7,8-tétrahydro-5,5,8,8-tétraméthyl-2-naphtyl)acétamido]benzoïque.

### (a) syn-4-[α-hydroxyheptyloxyimino-(5,6,7,8-tétrahydro-5,5,8,8-tétraméthyl-2-naphtyl)acétamido]benzoate d'allyle.

De manière analogue à l'exemple 1(f) par réaction de 2,2 g (5 mmoles) de 4-[α-hydroxyimino-(5,6,7,8-tétrahydro-5,5,8,8-tétraméthyl-2-naphtyl)acétamido] benzoate d'allyle avec 780 µl (5 mmoles) de 7-bromo-1-heptanol, on recueille après purification par chromatographie sur colonne de silice élué avec du dichlorométhane 1,7 g (61%) de l'ester allylique attendu sous forme d'une huile incolore.

### (b) acide syn-4-[α-hydroxyheptyloxyimino-(5,6,7,8-tétrahydro-5,5,8,8-tétraméthyl-2-naphtyl)acétamido]benzoïque.

De manière analogue à l'exemple 2, à partir de 1,7 g (3,1 mmoles) de syn-4-[α-hydroxyheptyloxyimino-(5,6,7,8-tétrahydro-5,5,8,8-tétraméthyl-2-naphtyl)acétamido] benzoate d'allyle on obtient 1,3 g (82%) de l'acide attendu de point de fusion 145-55°C.

### EXEMPLE 9

### Acide trans-4-[α-hydroxyheptyloxyimino-(5,6,7,8-tétrahydro-5,5,8,8-tétraméthyl-2-naphtyl)acétamido]benzoïque.

De manière analogue à l'exemple 3 à partir de 900 mg (1,77 mmole) d'acide syn-4-[α-hydroxyheptyloxyimino-(5,6,7,8-tétrahydro-5,5,8,8-tétraméthyl-2-naphtyl) acétamido]benzoïque, on obtient 350 mg (39%) de l'acide trans attendu de point de fusion 118-23°C.

### EXEMPLE 10

### Acide syn-4-[α-carboxypentyloxyimino-(5,6,7,8-tétrahydro-5,5,8,8-tétraméthyl-2-naphtyl)acétamido]benzoïque.

Dans un ballon, on introduit 1,3 g (2,4 mmoles) d'acide *syn-4-[α-éthoxy* carbonylpentyloxyimino-(5,6,7,8-tétrahydro-5,5,8,8-tétraméthyl-2-naphtyl)acétamido] benzoïque 30 ml de THF et ajoute 6 ml d'une solution de soude méthanolique 2N. On agite à température ambiante pendant trois heures, évapore à sec, reprend le résidu par l'eau, acidifie à pH 1 avec de l'acide chlorhydrique, extrait avec de l'éther éthylique. On décante la phase organique, sèche sur sulfate de magnésium, évapore. Ler résidu obtenu est purifié par chromatographie sur colonne de silice élué avec un mélange de dichlorométhane et d'éther éthylique (95-5). Après évaporation des solvants, on recueille 1,1 g (89%) de l'acide attendu de point de fusion 162-3°C.

### EXEMPLE 11

### Acide trans-4-[α-hydroxypropyloxyimino-(5,6,7,8-tétrahydro-5,5,8,8-tétraméthyl-2-naphtyl)acétamido/benzoïque.

### (a) 4-[α-hydroxypropyloxyimino-(5,6,7,8-tétrahydro-5,5,8,8-tétraméthyl-2-naphtyl)acétamido]benzoate d'allyle.

De manière analogue à l'exemple 1(f) par réaction de 3,4 g (7,8 mmoles) de 4-[α-hydroxyimino-(5,6,7,8-tétrahydro-5,5,8,8-tétraméthyl-2-naphtyl)acétamido] benzoate d'allyle avec 710 µl (7,8 mmoles) de 3-bromo-1-propanol, on recueille après purification par chromatographie sur colonne de silice élué avec du dichlorométhane 1,4 g (36%) de l'ester allylique syn et 710 mg (18%) de l'ester allylique trans.

### (b) acide trans-4-[α-hydroxypropyloxyimino-(5,6,7,8-tétrahydro-5,5,8,8-tétraméthyl-2-naphtyl)acétamido]benzoïque.

De manière analogue à l'exemple 2, à partir de 710 mg (1,4 mmole) de trans-4-[α-hydroxypropyloxyimino-(5,6,7,8-tétrahydro-5,5,8,8-tétraméthyl-2-naphtyl) acétamido] benzoate d'allyle on obtient 220 mg (35%) de l'acide attendu de point de fusion 209-11°C.

### EXEMPLE 12

### Acide syn-4-[α-hydroxypropyloxyimino-(5,6,7,8-tétrahydro-5,5,8,8-tétraméthyl-2-naphtyl)acétamido]benzoïque.

De manière analogue à l'exemple 2, à partir de 1,4 g (2,8 mmoles) de syn-4-[α-hydroxypropyloxyimino-(5,6,7,8-tétrahydro-5,5,8,8-tétraméthyl-2-naphtyl) acétamido] benzoate d'allyle on obtient 750 mg (58%) de l'acide attendu de point de fusion 198-204°C.

### EXEMPLE 13

### Acide syn-4-[α-éthoxycarbonylpropyloxyimino-(5,6,7,8-tétrahydro-5,5,8,8-tétraméthyl-2-naphtyl)acétamido]benzoïque.

### (a) syn-4-[α-éthoxycarbonylpropyloxyimino-(5,6,7,8-tétrahydro-5,5,8,8-tétraméthyl-2-naphtyl)acétamido]benzoate d'allyle.

De manière analogue à l'exemple 1(f) par réaction de 4 g (9,2 mmoles) de 4-[α-hydroxyimino-(5,6,7,8-tétrahydro-5,5,8,8-tétraméthyl-2-naphtyl)acétamido] benzoate d'allyle avec 1,8 g (9,2 mmoles) de 4-bromobutanoate d'éthyle on recueille après purification par chromatographie sur colonne de silice élué avec un mélange de dichlorométhane et d'heptane (50-50) 1,3 g (26%) de l'ester allylique attendu sous forme d'une huile incolore.

### (b) acide syn-4-[α-éthoxycarbonylpropyloxyimino-(5,6,7,8-tétrahydro-5,5,8,8-tétraméthyl-2-naphtyl)acétamido]benzoïque.

De manière analogue à l'exemple 2, à partir de 1,4 g (2,5 mmoles) de syn-4-[α-éthoxycarbonylpropyloxyimino-(5,6,7,8-tétrahydro-5,5,8,8-tétraméthyl-2-naphtyl)acétamido] benzoate d'allyle on obtient 180 mg (14%) de l'acide attendu de point de fusion 120-1°C.

### EXEMPLE 14

### Acide syn-4-[α-hydroxydecyloxyimino-(5,6,7,8-tétrahydro-5,5,8,8-tétraméthyl-2-naphtyl)acétamido]benzoïque.

### (a) syn-4-[α-hydroxydecyloxyimino-(5,6,7,8-tétrahydro-5,5,8,8-tétraméthyl-2-naphtyl)acétamido]benzoate d'allyle.

De manière analogue à l'exemple 1(f) par réaction de 2,2 g (5 mmoles) de 4-[α-hydroxyimino-(5,6,7,8-tétrahydro-5,5,8,8-tétraméthyl-2-naphtyl)acétamido] benzoate d'allyle avec 995 µl (5 mmoles) de 10-bromo-1-décanol, on recueille après purification par chromatographie sur colonne de silice élué avec du dichlorométhane 1 g (35%) de l'ester allylique attendu sous forme d'une huile incolore.

### (b) acide syn-4-[α-hydroxydecyloxyimino-(5,6,7,8-tétrahydro-5,5,8,8-tétraméthyl-2-naphtyl)acétamido]benzoïque.

De manière analogue à l'exemple 2, à partir de 1 g (1,7 mmole) de syn-4-[α-hydroxydecyloxyimino-(5,6,7,8-tétrahydro-5,5,8,8-tétraméthyl-2-naphtyl)acétamido] benzoate d'allyle on obtient 400 mg (50%) de l'acide attendu de point de fusion 145-6°C.

### EXEMPLE 15

### Acide syn-4-[α-hydroxynonyloxyimino-(5,6,7,8-tétrahydro-5,5,8,8-tétraméthyl-2-naphtyl)acétamido]benzoïque.

### (a) syn-4-[α-hydroxynonyloxyimino-(5,6,7,8-tétrahydro-5,5,8,8-tétraméthyl-2-naphtyl)acétamido]benzoate d'allyle.

De manière analogue à l'exemple 1(f) par réaction de 2,2 g (5 mmoles) de 4-[α-hydroxyimino-(5,6,7,8-tétrahydro-5,5,8,8-tétraméthyl-2-naphtyl)acétamido] benzoate d'allyle avec 780 µl (5 mmoles) de 7-bromo-1-heptanol, on recueille après purification par chromatographie sur colonne de silice élué avec du dichlorométhane 1,7 g (61%) de l'ester allylique attendu sous forme d'une huile incolore.

### (b) acide syn-4-[α-hydroxynonyloxyimino-(5,6,7,8-tétrahydro-5,5,8,8-tétraméthyl-2-naphtyl)acétamido]benzoïque.

De manière analogue à l'exemple 2, à partir de 1,,3 g (2,2 mmoles) de syn-4-[α-hydroxynonyloxyimino-(5,6,7,8-tétrahydro-5,5,8,8-tétraméthyl-2-naphtyl) acétamido] benzoate d'allyle on obtient 600 mg (50%) de l'acide attendu de point de fusion 155-6°C.

### EXEMPLE 16

### Acide syn-4-[α-benzyloxyimino-(5,6,7,8-tétrahydro-5,5,8,8-tétraméthyl-2-naphtyl)acétamido]benzoïque.

### (a) syn-4-[α-benzyloxyimino-(5,6,7,8-tétrahydro-5,5,8,8-tétraméthyl-2-naphtyl)acétamido]benzoate d'allyle.

De manière analogue à l'exemple 1(f) par réaction de 2,2 g (5 mmoles) de 4-[α-hydroxyimino-(5,6,7,8-tétrahydro-5,5,8,8-tétraméthyl-2-naphtyl)acétamido] benzoate d'allyle avec 600 µl (5 mmoles) de bromure de benzyle, on recueille après purification par chromatographie sur colonne de silice élué un mélange de dichlorométhane et d'heptane (60-40) 2,6 g (98%) de l'ester allylique attendu sous forme d'une huile incolore.

### (b) acide syn-4-[α-benzyloxyimino-(5,6,7,8-tétrahydro-5,5,8,8-tétraméthyl-2-naphtyl)acétamido]benzoïque.

De manière analogue à l'exemple 2, à partir de 2,6 g (5 mmoles) de syn-4-[α-benzyloxyimino-(5,6,7,8-tétrahydro-5,5,8,8-tétraméthyl-2-naphtyl)acétamido] benzoate d'allyle on obtient 1,7 g (71%) de l'acide attendu de point de fusion 223-5°C.

### EXEMPLE 17

### Acide syn-4-[α-methoxyethoxymethoxyimino-5,6,7,8-tétrahydro-5,5,8,8-tétraméthyl-2-naphthyl)acétamido]benzoïque.

### (a) syn-4-[α-methoxyethoxymethoxyimino-(5,6,7,8-tétrahydro-5,5,8,8-tétraméthyl-2-naphtyl)acétamido]benzoate d'allyle.

De manière analogue à l'exemple 1(f) par réaction de 3,3 g (7,6 mmoles) de 4-[α-hydroxyimino-(5,6,7,8-tétrahydro-5,5,8,8-tétraméthyl-2-naphtyl)acétamido] benzoate d'allyle avec 870 µl (7,6 mmoles) de chlorure de méthoxyethoxyméthane on recueille après purification par chromatographie sur colonne de silice élué avec un mélange de dichlorométhane et d'heptane (70-30) 1,2 g (30%) de l'ester allylique attendu sous forme d'une huile marron.

### (b) acide syn-4-[α-methoxyethoxymethoxyimino-5,6,7,8-tétrahydro-5,5,8,8-tétraméthyl-2-naphtyl)acétamido]benzoïque.

De manière analogue à l'exemple 2, à partir de 1,1 g (2,1 mmoles) de syn-4-[α-methoxyethoxymethoxyimino-(5,6,7,8-tétrahydro-5,5,8,8-tétraméthyl-2-naphtyl)acétamido] benzoate d'allyle on obtient 780 mg (77%) de l'acide attendu de point de fusion 113-15°C.

### EXEMPLE 18

### syn-4-[α-éthoxycarbonylpropyloxyimino-5,6,7,8-tétrahydro-5,5,8,8-tétraméthyl-2-naphyl)acétamido]benzoate d'allyle.

De manière analogue à l'exemple 1(f) par réaction de 3,9 g (9 mmoles) de 4-[α-hydroxyimino-(5,6,7,8-tétrahydro-5,5,8,8-tétraméthyl-2-naphtyl)acétamido] benzoate d'allyle avec 2,8 g (9 mmoles) de 8-bromooctanoate d'éthyle, on recueille après purification par chromatographie sur colonne de silice élué avec du dichlorométhane 2,4 g (50%) de l'ester allylique attendu sous forme d'une huile incolore.

### EXEMPLE 19

### Acide (Z)-4-[2-(5,6,7,8-tétrahydro-5,5,8,8-tétraméthyl-2-naphtyl)-2-nonenamido]benzoïque.

### (a) acide 2-(5,6,7,8-tétrahydro-5,5,8,8-tétraméthyl-2-naphtyl)-2-nonenoïque.

Dans un tricol et sous courant d'azote, on introduit 1 g (3,8 mmoles) d'acide 5,6,7,8-tétrahydro-5,5,8,8-tétraméthyl-2-naphtylglyoxylique et 10 ml de DMSO. On ajoute goutte à goutte 2,37 ml de n-BuLi (1,6 M dans l'hexane) et agite à température ambiante 15'. On ajoute ensuite 2 g (4,5 mmoles) de bromure de n-heptyltriphénylphosphine et additionne goutte à goutte 2,85 ml (4,5 mmoles) de n-BuLi (1,6 M dans l'hexane) et agite à température ambiante pendant une heure. On verse le milieu réactionnel dans l'eau, ajuste à pH 1 avec de l'acide chlorhydrique, extrait avec de l'éther éthylique, décante la phase organique, sèche sur sulfate de magnésium, évapore.Le résidu obtenu est purifié par chromatographie sur colonne de silice élué avec un mélange d'éther éthylique et d'heptane (40-60). On recueille après évaporation des solvants 380 mg (28%) du produit attendu sous forme d'une huile.

### (b) Chlorure de 2-(5,6,7,8-tétrahydro-5,5,8,8-tétraméthyl-2-naphtyl)-2-nonenoyle.

Dans un ballon on introduit 1,7 g (5 mmoles) d'acide 2-(5,6,7,8-tétrahydro-5,5,8,8-tétraméthyl-2-naphtyl)-2-nonenoïque 20 ml de toluène et 73µl de DMF. On chauffe à 50°C et ajoute 714 µl (6 mmoles) de chlorure de thionyle et agite à cette température pendant une heure. On évapore à sec et utilisera le chlorure d'acide brut pour la suite de la synthese.

### (c) (Z) et (E) -4-[2-(5,6,7,8-tétrahydro-5,5,8,8-tétraméthyl-2-naphtyl)-2-nonenamido]benzoate de méthyle.

Dans un tricol et sous courant d'azote on introduit 830 mg (5,5 mmoles) de 4-aminobenzoate de méthyle 20 ml de THF et 612 µl (6 mmoles) de triéthylamine. On ajoute goutte à goutte le chlorure d'acide préparé précedemment dissous dans 10 ml de THF et agite à température ambiante pendant huit heures. On verse le milieu réactionnel dans l'eau, extrait avec de l'éther éthylique, décante la phase organique, sèche sur sulfate de magnésium, évapore. On recueille un mélange des deux isomères géométriques que l'on sépare par chromatographie sur colonne de silice élué avec un mélange de dichlorométhane et d'heptane (65-35).

On recueille:
- 1 g (42%) de (Z)-4-[2-(5,6,7,8-tétrahydro-5,5,8,8-tétraméthyl-2-naphtyl)-2-nonenamido]benzoate de méthyle de point de fusion 129-30°C.
- 400 mg (18%) de (E) 4-[2-(5,6,7,8-tétrahydro-5,5,8,8-tétraméthyl-2-naphtyl)-2-nonenamido]benzoate de méthyle de point de fusion 86-7°C.

### (d) acide (Z)-4-[2-(5,6,7,8-tétrahydro-5,5,8,8-tétraméthyl-2-naphtyl)-2-nonenamido]benzoïque.

Dans un ballon on introduit 700 mg (1,47 mmole) de (Z) -4-[2-(5,6,7,8-tétrahydro-5,5,8,8-tétraméthyl-2-naphtyl)-2-nonenamido]benzoate de méthyle 25 ml de méthanol et 1,2 ml (14,7 mmoles) d'une solution d'hydroxyde de sodium à 35%. On agite à température ambiante pendant 12 heures, verse le milieu réactionnel dans l'eau, acidifie à pH 1 avec de l'acide chlorhydrique (37%), extrait avec de l'éther éthylique, décante la phase organique, sèche sur sulfate de magnésium, évapore. Le résidu est trituré dans l'heptane, filtré, séché. On recueille 555 mg (82%) d'acide (Z)-4-[2-(5,6,7,8-tétrahydro-5,5,8,8-tétraméthyl-2-naphtyl)-2-nonenamido]benzoïque de point de fusion 183-4°C.

### EXEMPLE 20

### Acide(E)-4-[2-(5,6,7,8-tétrahydro-5,5,8,8-tétraméthyl-2-naphthyl)-2-hexadecenamido]benzoique.

### (a) acide 2-(5,6,7,8-tétrahydro-5,5,8,8-tétraméthyl-2-naphtyl)-2-hexadecenoïque.

De manière analogue à l'exemple 19(a) par réaction de 9,65 g (37,1 mmoles) d'acide 5,6,7,8-tétrahydro-5,5,8,8-tétraméthyl-2-naphtylglyoxylique avec 24 g (44,5 mmoles) de bromure de tétradecyltriphénylphosphine, on obtient après purification sur colonne de silice élué avec du dichlorométhane 4,53 g (28%) du produit attendu sous forme d'une huile qui cristallise lentement point de fusion 59-60°C.

### (b) chlorure de 2-(5,6,7,8-tétrahydro-5,5,8,8-tétraméthyl-2-naphtyl)-2-hexadecenoyle.

De manière analogue à l'exemple 19 (b) par réaction de 2 g (4,5 mmoles) d'acide 2-(5,6,7,8-tétrahydro-5,5,8,8-tétraméthyl-2-naphtyl)-2-hexadecenoïque avec 648 µl (5,4 mmoles) de chlorure de thionyle, on obtient le chlorure d'acide qui sera utilisé brut pour la suite de la synthèse.

### (c) (Z)4-[2-(5,6,7,8-tétrahydro-5,5,8,8-tétraméthyl-2-naphtyl)-2-hexadecenamido] benzoate de méthyle.

De manière analogue à l'exemple 19 (c) par réaction du chlorure d'acide obtenu précedemment avec 755 mg (5 mmoles) de 4-aminobenzoate de méthyle, on obtient après chromatographie sur colonne de silice élué avec un mélange de dichlorométhane et d'heptane (50-50) 1,16 g (42%) de (Z)-4-[2-(5,6,7,8-tétrahydro-5,5,8,8-tétraméthyl-2-naphtyl)-2-hexadecenamido]benzoate de méthyle de point de fusion 93-4°C.

### (d) acide (Z)4-[2-(5,6,7,8-tétrahydro-5,5,8,8-tétraméthyl-2-naphtyl)-2-hexadecenamido]benzoïque.

De manière analogue à l'exemple 19 (d) à partir de 503 mg (0,9 mmole) de (Z)-4-[2-(5,6,7,8-tétrahydro-5,5,8,8-tétraméthyl-2-naphtyl)-2-hexadecenamido] benzoate de méthyle, on obtient 435 mg (89%) d'acide (Z)-4-[2-(5,6,7,8-tétrahydro-5,5,8,8-tétraméthyl-2-naphyl)-2-hexadecenamido]benzoïque de point de fusion 164-5°C.

### EXEMPLE 21

Dans cet exemple, on a illustré diverses formulations concrètes à base des composés selon l'invention.

### A- VOIE ORALE

| (a) Comprimé de 0,2 g | |
|---|---|
| - Composé préparé à l'exemple 3 | 0,001 g |
| - Amidon | 0,114 g |
| - Phosphate bicalcique | 0,020 g |
| - Silice | 0,020 g |
| - Lactose | 0,030 g |
| - Talc | 0,010 g |
| - Stéarate de magnésium | 0,005 g |

| (b) Suspension buvable en ampoules de 5 ml | |
|---|---|
| - Composé préparé à l'exemple 4 | 0,001 g |
| - Glycérine | 0,500 g |
| - Sorbitol à 70% | 0,500 g |
| - Saccharinate de sodium | 0,010 g |
| - Parahydroxybenzoate de méthyle | 0,040 g |
| - Arome qs | |
| - Eau purifiée qsp | 5 ml |

### B- VOIE TOPIQUE

| (a) Onguent | |
|---|---|
| - Composé de l'exemple 3 | 0,020 g |
| - Myristate d'isopropyle | 81,700 g |
| - Huile de vaseline fluide | 9,100 g |
| - Silice ("Aérosil 200" vendue par DEGUSSA) | 9,180 g |

| (b) Onguent | |
|---|---|
| - Composé de l'exemple 5 | 0,300 g |
| - Vaseline blanche codex | 100 g |

| (c) Crème Eau-dans-Huile non ionique | |
|---|---|
| - Composé de l'exemple 4 | 0,100 g |
| - Mélange d'alcools de lanoline émulsifs, de cires et d'huiles ("Eucerine anhydre" vendu par BDF) | 39,900 g |
| - Parahydroxybenzoate de méthyle | 0,075 g |
| - Parahydroxybenzoate de propyle | 0,075 g |
| - Eau déminéralisée stérile qsp | 100 g |

| (d) Lotion | |
|---|---|
| - Composé de l'exemple 5 | 0,100 g |
| - Polyéthylène glycol (PEG 400) | 69,900 g |
| - Ethanol à 95% | 30,000 g |

| (e) Onguent hydrophobe | |
|---|---|
| - Composé de l'exemple 2 | 0,300 g |
| - Mirystate d'isopropyle | 36,400 g |
| - Huile de silicone ("Rhodorsil 47 V 300" vendu par RHONE-POULENC) | 36,400 g |
| - Cire d'abeille | 13,600 g |
| - Huile de silicone ("Abil 300.000 cst" vendu par GOLDSCHMIDT) | 100g |

| (f) Crème Huile-dans-Eau non ionique | |
|---|---|
| - Composé de l'exemple 3 | 1,000 g |
| - Alcool cétylique | 4,000 g |
| - Monostéarate de glycérole | 2,500 g |
| - Stéarate de PEG 50 | 2,500 g |
| - Beurre de karité | 9,200 g |
| - Propylène glycol | 2,000 g |
| - Parahydroxybenzoate de méthyle | 0,075 g |
| - Parahydroxybenzoate de propyle | 0,075 g |
| - Eau déminéralisée stérile | 100 g |

## Revendications

1. Composés bi-aromatiques dérivés d'amide, caractérisés par le fait qu'ils répondent à la formule générale (I) suivante : 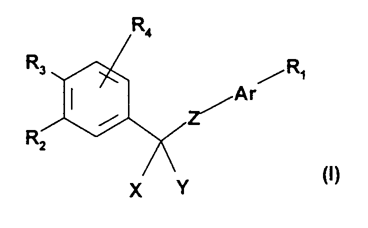 dans laquelle :
∗ Ar représente un radical choisi parmi les radicaux de formules (a)-(e) suivantes: 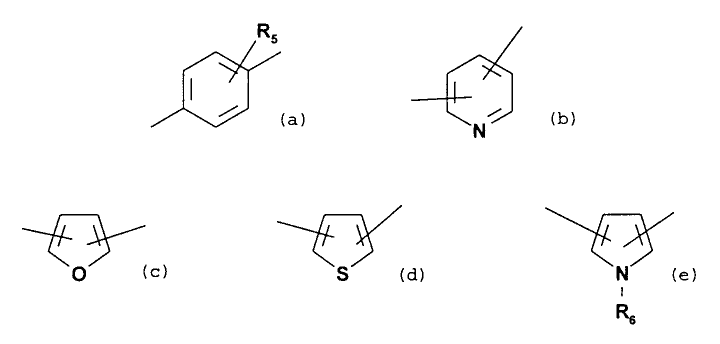
R₅ et R₆ ayant les significations données ci-après,
∗ Z représente -CO-NR₇-,
∗ R₁ représente :
(i) un radical -CH₃
(ii) un radical -CH₂-O-R₆
(iii) un radical -O-R₆
(iv) un radical -CO-R₈,
(v) un radical -S(O)ₜR₉
R₆, R₈, R₉ et t ayant les significations données ci-après,
* soit X et Y peuvent être pris séparement :
X représente un atome d'hydrogène ou un radical alkyle inférieur de C₁ à C₆,
Y représente un radical de formule -(CH₂)ₙ-CH₃ ou de formule -(CH₂)ₙ-R₁₀
* soit X et Y peuvent être pris ensemble :
X et Y forment un radical unique à double liaison choisi parmi les radicaux de formules :
-
=N-O-(CH₂)ₙ-R₁₀
-
=CH-(CH₂)ₘ-R₁₀
-
=N-O-(CH₂)₂-O-CH₂-CH₃
-
=N-O-CH₂-O-CH₂-CH₂-O-CH₃
-
=CH-(CH₂)ₘ-CH₃
R₁₀, m et n ayant les significations données ci-après,
∗ R₂ et R₃, pris séparément, représentent un atome d'hydrogène, un radical alkyle linéaire ou ramifié ayant de 1 à 20 atomes de carbone, un radical -OR₆ ou un radical -SR₆, R₆ ayant la signification donnée ci-après, étant entendu que R₂ et R₃ ne peuvent pas être simultanément un atome d'hydrogène, un radical -OR₆ ou un radical -SR₆, ou R₂ et R₃, pris ensemble, forment avec le cycle aromatique adjacent un cycle à 5 ou 6 chainons éventuellement substitué par des groupes méthyle et/ou éventuellement interrompu par un atome d'oxygène ou de soufre ou par un radical -SO- ou SO₂-,
∗ R₄ représente un atome d'hydrogène, un atome d'halogène, un radical alkyle inférieur de C₁ à C₆ ou un radical -OR₆, R₆ ayant la signification donnée ci-après,
étant entendu que dans tout ce qui précède :
- R₅ a la même signification que R₄,
- R₆ représente un atome d'hydrogène, un radical alkyle linéaire ou ramifié ayant de 1 à 20 atomes de carbone ou un radical -CO-R₁₁, R₁₁ ayant la signification donnée ci-après,
- R₇ représente un atome d'hydrogène ou un radical alkyle inférieur de C₁ à C₆,
- R₈ représente :
(a) un atome d'hydrogène
(b) un radical alkyle inférieur de C₁ à C₆
(c) un radical de formule : 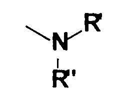 R' et R'' ayant les significations données ci-après,
(d) un radical -OR₁₂, R₁₂ ayant la signification donnée ci-après,
- R₉ et R₁₁, identiques ou différents, représentent un radical alkyle inférieur de C₁ à C₆,
- R₁₀ représente :
(i) un radical -OR₆
(ii) un radical -CO-R₈
(iii) un radical de formule 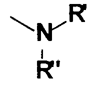 R' et R'' ayant les significations données ci-dessous,
(iv) un radical aryle, éventuellement substitué,
(v) un radical hétérocycle,
(vi) un radical cycloaliphatique en C₃ ou C₆,
- R₁₂ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,
- R' et R'', identiques ou différents, représentent un atome d'hydrogène, un radical alkyle inférieur de C₁ à C₆, un radical mono ou polyhydroxyalkyle, un radical aryle éventuellement substitué ou un reste d'aminoacide ou de peptide ou de sucre ou encore, pris ensemble, forment un hétérocycle,
- m est un nombre entier de 1 à 20,
- n est un nombre entier de 2 à 20,
- t est un nombre entier égal à 0,1 ou 2,
ainsi que leurs sels, et leurs isomères optiques et géométriques.

2. Composés selon la revendication 1, caractérisés en ce qu'ils se présentent sous forme de sels d'un métal alcalin ou alcalino-terreux, ou encore de zinc ou d'une amine organique.

3. Composés selon l'une des revendications 1 ou 2, caractérisés en ce que les radicaux alkyles inférieurs sont choisis dans le groupe constitué par les radicaux méthyle, éthyle, isopropyle, butyle, tertiobutyle et hexyle.

4. Composés selon l'une quelconque des revendications précédentes, caractérisés en ce que les radicaux alkyles linéaires ou ramifiés ayant de 1 à 15 atomes de carbone sont choisis dans le groupe constitué par les radicaux méthyle, éthyle, propyle, 2-éthyl-hexyle, octyle, dodécyle, hexadécyle et octadécyl.

5. Composés selon l'une quelconque des revendications précédentes, caractérisés en ce que les radicaux monohydroxyalkyles sont choisis dans le groupe constitué par les radicaux 2-hydroxyéthyle, 2-hydroxypropyle ou 3-hydroxypropyle.

6. Composés selon l'une quelconque des revendications précédentes, caractérisés en ce que les radicaux polyhydroxyalkyles sont choisis dans le groupe constitué par les radicaux 2,3-dihydroxypropyle, 2,3,4-trihydroxybutyle, 2,3,4,5-tétrahydroxypentyle ou le reste du pentaérythritol.

7. Composés selon l'une quelconque des revendications précédentes, caractérisés en ce que le radical aryle est un radical phényle éventuellement substitué par au moins un atome d'halogène, un hydroxyle ou une fonction nitro.

8. Composés selon l'une quelconque des revendications précédentes, caractérisés en ce que les radicaux aralkyles sont choisis dans le groupe constitué par les radicauxl benzyle ou phénéthyle éventuellement substitués par au moins un atome d'halogène, un hydroxyle ou une fonction nitro.

9. Composés selon l'une quelconque des revendications précédentes, caractérisés en ce que les radicaux alkényles sont choisis dans le groupe constitué par les radicaux contenant de 1 à 5 atomes de carbone et présentant une ou plusieurs insaturations éthyléniques, en particulier le radical allyle.

10. Composés selon l'une quelconque des revendications précédentes, caractérisés en ce que les restes de sucre sont choisis dans le groupe constitué par les restes de glucose, de galactose, de mannose ou d'acide glucuronique.

11. Composés selon l'une quelconque des revendications précédentes, caractérisés en ce que les restes d'aminoacide sont choisis dans le groupe constitué par les restes dérivant de la lysine, de la glycine ou de l'acide aspartique.

12. Composés selon l'une quelconque des revendications précédentes, caractérisés en ce que les restes de peptides sont choisis dans le groupe constitué par les restes de dipeptides ou de tripeptides.

13. Composés selon l'une quelconque des revendications précédentes, caractérisés en ce que les radicaux hétérocycliques sont choisis dans le groupe constitué par les radicaux pipéridino, morpholino, pyrrolidino ou pipérazino, éventuellement substitués en position 4 par un radical alkyle en C₁-C₆ ou mono ou polyhydroxyalkyle.

14. Composés selon l'une quelconque des revendications précédentes, caractérisés en ce que les atomes d'halogènes sont choisis dans le groupe constitué par le fluor, le chlore et le brome.

15. Composés selon la revendication 1, caractérisés par le fait qu'ils sont pris dans le groupe constitué par:
- Acide *anti*-4-[α-hydroxyhexylimino-(5,6,7,8-tétrahydro-5,5,8,8-tétraméthyl-2-naphtyl)acétamido]benzoïque
- Acide *syn*-4-[α-hydroxyhexylimino-(5,6,7,8-tétrahydro-5,5,8,8-tétraméthyl-2-naphtyl)acétamido]benzoïque
- Acide *syn*-4-[α-méthoxycarbonylpentylimino-(5,6,7,8-tétrahydro-5,5,8,8-tétraméthyl-2-naphtyl)acétamido]benzoïque
- Acide syn-4-[α-hydroxyoctyloxyimino-(5,6,7,8-tétrahydro-5,5,8,8-tétraméthyl-2-naphtyl)acétamido]benzoïque.
- Acide trans-4-[α-hydroxyoctyloxyimino-(5,6,7,8-tétrahydro-5,5,8,8-tétraméthyl-2-naphtyl)acétamido]benzoïque.
- Acide syn-4-[α-hydroxyheptyloxyimino-(5,6,7,8-tétrahydro-5,5,8,8-tétraméthyl-2-naphtyl)acétamido]benzoïque.
- Acide trans-4-[α-hydroxyheptyloxyimino-(5,6,7,8-tétrahydro-5,5,8,8-tétraméthyl-2-naphtyl)acétamido]benzoïque.
- Acide syn-4-[α-hydroxypropyloxyimino-(5,6,7,8-tétrahydro-5,5,8,8-tétraméthyl-2-naphtyl)acétamido]benzoïque.
- Acide trans-4-[α-hydroxypropyloxyimino-(5,6,7,8-tétrahydro-5,5,8,8-tétraméthyl-2-naphtyl)acétamido]benzoïque.
- Acide syn-4-[α-éthoxycarbonylpropyloxyimino-(5,6,7,8-tétrahydro-5,5,8,8-tétraméthyl-2-naphtyl)acétamido]benzoïque.
- Acide syn-4-[α-hydroxydecyloxyimino-(5,6,7,8-tétrahydro-5,5,8,8-tétraméthyl-2-naphtyl)acétamido]benzoïque.
- Acide syn-4-[α-hydroxynonyloxyimino-(5,6,7,8-tétrahydro-5,5,8,8-tétraméthyl-2-naphtyl)acétamido]benzoïque.
- Acide syn-4-[α-benzyloxyimino-(5,6,7,8-tétrahydro-5,5,8,8-tétraméthyl-2-naphtyl)acétamido]benzoïque.
- Acide syn-4-[α-methoxyethoxymethoxyimino-(5,6,7,8-tétrahydro-5,5,8,8-tétraméthyl-2-naphtyl)acétamido]benzoïque.
- syn-4-[α-éthoxycarbonylpropyloxyimino-(5,6,7,8-tétrahydro-5,5,8,8-tétraméthyl-2-naphtyl)acétamido]benzoate d'allyle.
- Acide (E)-4-[2-(5,6,7,8-tétrahydro-5,5,8,8-tétraméthyl-2-naphtyl)-2-nonenamido]benzoïque.
- Acide (Z)-4-[2-(5,6,7,8-tétrahydro-5,5,8,8-tétraméthyl-2-naphtyl)-2-nonenamido]benzoïque.
- Acide (E)-4-[2-(5,6,7,8-tétrahydro-5,5,8,8-tétraméthyl-2-naphyl)-2-undecenamido]benzoïque.
- (E)4-[2-(5,6,7,8-tétrahydro-5,5,8,8-tétraméthyl-2-naphtyl)-2-undecenamido]phénylcarboxamide.
- (E)-4-[2-(5,6,7,8-tétrahydro-5,5,8,8-tétraméthyl-2-naphtyl)-2-undecenamido]benzèneméthanol.
- (E)-4-[2-(5,6,7,8-tétrahydro-5,5,8,8-tétraméthyl-2-naphtyl)-2-undecenamido]phénol.
- Acide (Z)-4-[2-(5,6,7,8-tétrahydro-5,5,8,8-tétraméthyl-2-naphtyl)-2-undecenamido]benzoïque.
- Acide(E)-4-[2-(5,6,7,8-tétrahydro-5,5,8,8-tétraméthyl-2-naphtyl)-2-hexadecenamido]benzoïque.
- Acide (Z)-4-[2-(5,6,7,8-tétrahydro-5,5,8,8-tétraméthyl-2-naphtyl)-2-hexadecenamido]benzoïque.

16. Composés selon la revendication 1, caractérisés en ce que les composés de formule (I) sont ceux pour lesquels R₁ représente le radical -COR₈, X et Y pris ensemble forment un radical choisi parmi les radicaux de formules suivantes :
-
=N-O-(CH2)n-R₁₀
-
=CH-(CH2)m-R₁₀
-
=N-O-(CH₂)₂-O-CH₂-CH₃
-
=N-O-CH₂-O-CH₂-CH₂-O-CH₃
-
=CH-(CH₂)ₘ-CH₃,
R₈, R₁₀, m et n ayant les significations données dans la revendication 1.

17. Composés selon l'une quelconque des revendications précédentes pour une utilisation comme médicament.

18. Composés selon la revendication 17 pour une utilisation comme médicament destiné au traitement des affections dermatologiques, rhumatismales, respiratoires, cardiovasculires et ophtalmologiques.

19. Utilisation de l'un au moins des composés définis aux revendications 1 à 16 pour la fabrication d'un médicament destiné au traitement des affections dermatologiques, rhumatismales, respiratoires, cardiovasculires et ophtalmologiques.

20. Composition pharmaceutique, caractérisée par le fait qu'elle comprend, dans un support pharmaceutiquement acceptable, au moins un des composés tels que définis à l'une quelconque des revendications 1 à 16.

21. Composition selon la revendication 20, caractérisée en ce que la concentration en composé(s) selon l'une des revendication 1 à 16 est comprise entre 0,001 % et 5 % en poids par rapport à l'ensemble de la composition.

22. Composition cosmétique, caractérisée par le fait qu'elle comprend, dans un support cosmétiquement acceptable, au moins un des composés tels que définis à l'une quelconque des revendications 1 à 16.

23. Composition selon la revendication 22, caractérisée en ce que la concentration en composé(s) selon l'une des revendications 1 à 16 est comprise entre 0,001 % et 3 % en poids par rapport à l'ensemble de la composition.

24. Utilisation d'une composition cosmétique telle que définie à l'une des revendications 22 ou 23 pour l'hygiène corporelle ou capillaire.

## Claims

1. Amide-derived biaromatic compounds, characterized by the fact that they correspond to the following general formula (I): 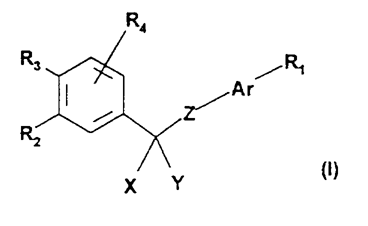 in which:
* Ar represents a radical chosen from the radicals of the following formulae (a)-(e) 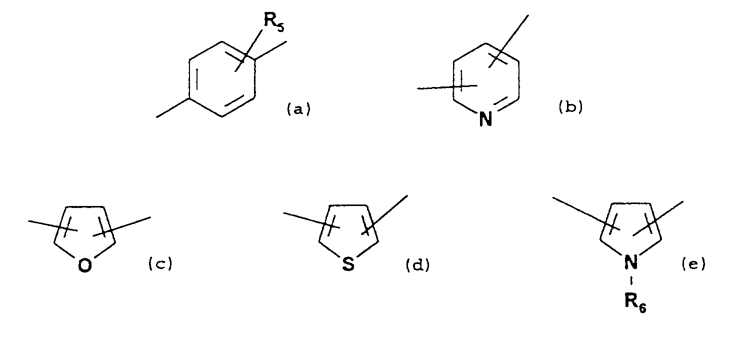
R₅ and R₆ having the meanings given below,
* Z represents -CO-NR₇-,
* R₁ represents:
(i) a -CH₃ radical
(ii) a radical -CH₂-O-R₆
(iii) a radical -O-R₆
(iv) a radical -CO-R₈
(v) a radical -s(O)ₜR₉
R₆, R₇, R₉ and t having the meanings given below,
* either X and Y may be taken separately:
X represents a hydrogen atom or a C₁ to C₆ lower alkyl radical,
Y represents a radical of formula -(CH₂)ₙ-CH₃ or of formula -(CH₂)ₙ-R₁₀
* or X and Y may be taken together:
X and Y form a single radical containing a double bond, chosen from the radicals of formulae:
-
=N-O-(CH₂)ₙ-R₁₀
-
=CH-(CH₂)ₘ-R₁₀
-
=N-O-(CH₂)₂-O-CH₂-CH₃
-
=N-O-CH₂-O-CH₂-CH₂-O-CH₃
-
=CH-(CH₂)ₘ-CH₃
R₁₀, m and n having the meanings given below,
* R₂ and R₃, taken separately, represent a hydrogen atom, a linear or branched alkyl radical having 1 to 20 carbon atoms, a radical -OR₆ or a radical -SR₆, R₆ having the meaning given below, it being understood that R₂ and R₃ cannot simultaneously be a hydrogen atom, a radical -OR₆ or a radical -SR₆, or R₂ and R₃, taken together, form with the adjacent aromatic ring a 5- or 6-membered ring optionally substituted by methyl groups and/or optionally interrupted by an oxygen or sulphur atom or by an -SO- or -SO₂- radical,
* R₄ represents a hydrogen atom, a halogen atom, a C₁ to C₆ lower alkyl radical or a radical -OR₆, R₆ having the meaning given below,
it being understood that in all that precedes:
- R₅ has the same meaning as R₄,
- R₆ represents a hydrogen atom, a linear or branched alkyl radical having 1 to 20 carbon atoms or a radical -CO-R₁₁, R₁₁ having the meaning given below,
- R₇ represents a hydrogen atom or a C₁ to C₆ lower alkyl radical,
- R₈ represents:
(a) a hydrogen atom
(b) a C₁ to C₆ lower alkyl radical
(c) a radical of formula: 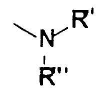 R' and R'' having the meanings given below,
(d) a radical -OR₁₂, R₁₂ having the meaning given below,
- R₉ and R₁₁, which may be identical or different, represent a C₁ to C₆ lower alkyl radical,
- R₁₀ represents:
(1) a radical -OR₆
(ii) a radical -CO-R₈
(iii) a radical of formula 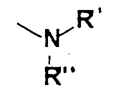 R' and R'' having the meanings given below,
(iv) an optionally substituted aryl radical,
(v) a heterocyclic radical,
(vi) a C₃ or C₆ cycloaliphatic radical,
- R₁₂ represents a hydrogen atom, a linear or branched alkyl radical having 1 to 20 carbon atoms, an alkenyl radical, a mono- or polyhydroxyalkyl radical, an optionally substituted aryl or aralkyl radical or a sugar residue or an amino acid or peptide residue,
- R' and R'', which may be identical or different, represent a hydrogen atom, a C₁ to C₆ lower alkyl radical, a mono or polyhydroxyalkyl radical, an optionally substituted aryl radical or an amino acid or peptide or sugar residue or alternatively, taken together, form a heterocycle,
- m is an integer from 1 to 20,
- n is an integer from 2 to 20,
- t is an integer equal to 0, 1 or 2.
as well as salts thereof, and optical and geometric isomers thereof.

2. Compounds according to Claim 1, characterized in that they are in the form of salts of an alkali or alkaline-earth metal, or alternatively of zinc or of an organic amine.

3. Compounds according to one of Claims 1 or 2, characterized in that the lower alkyl radicals are chosen from the group consisting of methyl, ethyl, isopropyl, butyl, tert-butyl and hexyl radicals.

4. Compounds according to any one of the preceding claims, characterized in that the linear or branched alkyl radicals having 1 to 15 carbon atoms are chosen from the group consisting of methyl, ethyl, propyl, 2-ethylhexyl, octyl, dodecyl, hexadecyl and octadecyl radicals.

5. Compounds according to any one of the preceding claims, characterized in that the monohydroxyalkyl radicals are chosen from the group consisting of 2-hydroxyethyl, 2-hydroxypropyl or 3-hydroxypropyl radicals.

6. Compounds according to any one of the preceding claims, characterized in that the polyhydroxyalkyl radicals are chosen from the group consisting of 2,3-dihydroxypropyl, 2,3,4-trihydroxybutyl or 2,3,4,5-tetrahydroxypentyl radicals or the pentaerythritol residue.

7. Compounds according to any one of the preceding claims, characterized in that the aryl radical is a phenyl radical optionally substituted by at least one halogen atom, a hydroxyl or a nitro functional group.

8. Compounds according to any one of the preceding claims, characterized in that the aralkyl radicals are chosen from the group consisting of benzyl or phenethyl radicals optionally substituted by at least one halogen atom, a hydroxyl or a nitro functional group.

9. Compounds according to any one of the preceding claims, characterized in that the alkenyl radicals are chosen from the group consisting of the radicals containing 1 to 5 carbon atoms and having one or more ethylenic unsaturations, in particular the allyl radical.

10. Compounds according to any one of the preceding claims, characterized in that the sugar residues are chosen from the group consisting of glucose, galactose, mannose or glucuronic acid residues.

11. Compounds according to any one of the preceding claims, characterized in that the amino acid residues are chosen from the group consisting of residues derived from lysine, glycine or aspartic acid.

12. Compounds according to any one of the preceding claims, characterized in that the peptide residues are chosen from the group consisting of dipeptide or tripeptide residues.

13. Compounds according to any one of the preceding claims, characterized in that the heterocyclic radicals are chosen from the group consisting of piperidino, morpholino, pyrrolidino or piperazino radicals which are optionally substituted at position 4 by a C₁-C₆ alkyl radical or a mono- or polyhydroxyalkyl radical.

14. Compounds according to any one of the preceding claims, characterized in that the halogen atoms are chosen from the group consisting of fluorine, chlorine and bromine.

15. Compounds according to Claim 1, characterized by the fact that they are chosen from the group consisting of;
- *anti*-4-[α-Hydroxyhexylimino-(5,6,7,8-tetrahydro-5,5,8,8-tetramethyl-2-naphthyl)acetamido]benzoic acid
- *syn*-4-[α-Hydroxyhexylimino-(5,6,7,8-tetrahydro-5,5,8,8-tetramethyl-2-naphthyl)acetamido]benzoic acid
- *syn*-4-[α-Methoxycarbonylpentylimino-(5,6,7,8-tetrahydro-5,5,8,8-tetramethyl-2-naphthyl)acetamido]benzoic acid
- *syn*-4-[α-Hydroxyoctyloxyimino-(5,6,7,8-tetrahydro-5,5,8,8-tetramethyl-2-naphthyl)acetamido]benzoic acid
- *trans*-4-[α-Hydroxyoctyloxyimino-(5,6,7,8-tetrahydro-5,5,8,8-tetramethyl-2-naphthyl)acetamido]benzoic acid
- *syn*-4-[α-Hydroxyheptyloxyimino-(5,6,7,8-tetrahydro-5,5,8,8-tetramethyl-2-naphthyl)acetamido]benzoic acid
- *trans*-4-[α-Hydroxyheptyloxyimino-(5,6,7,8-tetrahydro-5,5,8,8-tetramethyl-2-naphthyl)acetamido]benzoic acid
- *syn*-4-[α-Hydroxypropyloxyimino-(5,6,7,8-tetrahydro-5,5,8,8-tetramethyl-2-naphthyl)acetamido]benzoic acid
- *trans*-4-[α-Hydroxypropyloxyimino-(5,6,7,8-tetrahydro-5,5,8,8-tetramethyl-2-naphthyl)acetamido]benzoic acid
- *syn*-4-[α-Ethoxycarbonylpropyloxyimino-(5,6,7,8-tetrahydro-5,5,8,8-tetramethyl-2-naphthyl)acetamido]benzoic acid
- *syn*-4-[α-Hydroxydecyloxyimino-(5,6,7,8-tetrahydro-5,5,8,8-tetramethyl-2-naphthyl)acetamido]benzoic acid
- *syn*-4-[α-Hydroxynonyloxyimino-(5,6,7,8-tetrahydro-5,5,8,8-tetramethyl-2-naphthyl)acetamido]benzoic acid
- *syn*-4-[α-Benzyloxyimino-(5,6,7,8-tetrahydro-5,5,8,8-tetramethyl-2-naphthyl)acetamido]benzoic acid
- *syn*-4-[α-Methoxyethoxymethoxyimino-(5,6,7,8-tetrahydro-5,5,8,8-tetramethyl-2-naphthyl)acetamido]benzoic acid
- Allyl *syn*-4-[α-ethoxycarbonylpropyloxyimino(5,6,7,8-tetrahydro-5,5,8,8-tetramethyl-2-naphthyl)acetamido]benzoate
- (E)-4-[2-(5,6,7,8-Tetrahydro-5,5,8,8-tetramethyl-2-naphthyl)-2-nonenamido]benzoic acid
- (Z)-4-[2-(5,6,7,8-Tetrahydro-5,5,8,8-tetramethyl-2-naphthyl)-2-nonenamido]benzoic acid
- (E)-4-[2-(5,6,7,8-Tetrahydro-5,5,8,8-tetramethyl-2-naphthyl)-2-undecenamido]benzoic acid
- (E)-4-[2-(5,6,7,8-Tetrahydro-5,5,8,8-tetramethyl-2-naphthyl)-2-undecenamido]phenylcarboxamide
- (E)-4-[2-(5,6,7,8-Tetrahydro-5,5,8,8-tetramethyl-2-naphthyl)-2-undecenamido]benzenemethanol
- (E)-4-[2-(5,6,7,8-Tetrahydro-5,5,8,8-tetramethyl-2-naphthyl)-2-undecenamido]phenol
- (Z)-4-[2-(5,6,7,8-Tetrahydro-5,5,8,8-tetramethyl-2-naphthyl)-2-undecenamido]benzoic acid
- (E)-4-[2-(5,6,7,8-Tetrahydro-5,5,8,8-tetramethyl-2-naphthyl)-2-hexadecenamido]benzoic acid
- (Z)-4-[2-(5,6,7,8-Tetrahydro-5,5,8,8-tetramethyl-2-naphthyl)-2-hexadecenamido]benzoic acid.

16. Compounds according to Claim 1, characterized in that the compounds of formula (I) are those for which R₁ represents the radical -COR₈, X and Y, taken together, form a radical chosen from the radicals of following formulae:
-
=N-O-(CH₂)ₙ-R₁₀
-
=CH-(CH₂)ₘ-R₁₀
-
=N-O-(CH₂)₂-O-CH₂-CH₃
-
=N-O-CH₂-O-CH₂-CH₂-O-CH₃
-
=CH-(CH₂)ₘ-CH₃,
R₈, R₁₀, m and n having the meanings given in Claim 1.

17. Compounds according to any one of the preceding claims, for use as medicinal products.

18. Compounds according to Claim 17, for use as medicinal products intended for the treatment of dermatological, rheumatic, respiratory, cardiovascular and ophthalmological conditions.

19. Use of at least one of the compounds defined in Claims 1 to 16 for the manufacture of a medicinal product intended for the treatment of dermatological, rheumatic, respiratory, cardiovascular and ophthalmological conditions.

20. Pharmaceutical composition, characterized by the fact that it comprises, in a pharmaceutically acceptable carrier, at least one of the compounds as defined in any one of Claims 1 to 16.

21. Composition according to Claim 20, characterized in that the concentration of compound(s) according to one of Claims 1 to 16 is between 0.001 % and 5 % by weight relative to the whole composition.

22. Cosmetic composition, characterized by the fact that it comprises, in a cosmetically acceptable carrier, at least one of the compounds as defined in any one of Claims 1 to 16.

23. Composition according to Claim 22, characterized in that the concentration of compound(s) according to one of Claims 1 to 16 is between 0.001 % and 3 % by weight relative to the whole composition.

24. Use of a cosmetic composition as defined in one of Claims 22 or 23 for body or hair care.

## Patentansprüche

1. Von Amiden abgeleitete biaromatische Verbindungen, dadurch gekennzeichnet, daß sie der folgenden allgemeinen Formel (I) entsprechen: 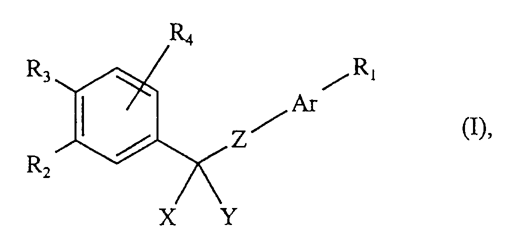 in der bedeuten:
• Ar eine Gruppe, die ausgewählt ist unter den Gruppen der folgenden Formeln (a) - (e): 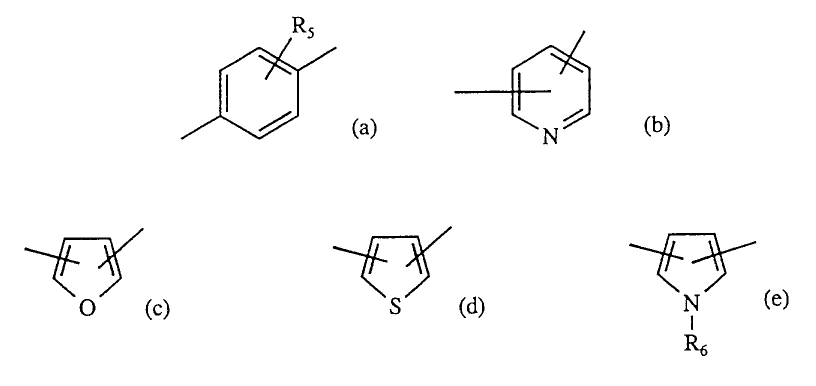
wobei R₅ und R₆ die unten angegebene Bedeutung besitzen,
• Z die Gruppe -CO-NR₇-,
• R₁:
(i) -CH₃,
(ii) eine Gruppe -CH₂-O-R₆,
(iii) eine Gruppe -O-R₆,
(iv) eine Gruppe -CO-R₈,
(v) eine Gruppe -S(O)ₜR₉,
wobei R₆, R₈, R₉ und t die unten angegebene Bedeutung besitzen,
• X und Y:
entweder unabhängig
X ein Wasserstoffatom oder eine niedere Alkylgruppe mit 1 bis 6 Kohlenstoffatomen und
Y eine Gruppe der Formel -(CH₂)ₙ-CH₃ oder der Formel -(CH₂)ₙ-R₁₀
oder zusammen
eine einzige, über eine Doppelbindung gebundene Gruppe, die ausgewählt ist unter den Gruppen der Formeln:
-
=N-O-(CH₂)ₙ-R₁₀,
-
=CH-(CH₂)ₘ-R₁₀,
-
=N-O-(CH₂)₂-O-CH₂-CH₃,
-
=N-O-CH₂-O-CH₂-CH₂-O-CH₃,
-
=CH-(CH₂)ₘ-CH₃,
wobei R₁₀, m und n die unten angegebene Bedeutung besitzen,
• R₂ und R₃
entweder unabhängig jeweils ein Wasserstoffatom, eine geradkettige oder verzweigte Alkylgruppe mit 1 bis 20 Kohlenstoffatomen, eine Gruppe -OR₆ oder eine Gruppe -SR₆, wobei R₆ die unten angegebene Bedeutung besitzt, mit der Maßgabe, daß R₂ und R₃ nicht gleichzeitig ein Wasserstoffatom, eine Gruppe -OR₆ oder eine Gruppe -SR₆ sein können,
oder
zusammen mit dem benachbarten aromatischen Fing einen 5- oder 6-gliedrigen Ring bilden, der gegebenenfalls mit Methylgruppen substituiert ist und/oder gegebenenfalls ein Sauerstoff- oder Schwefelatom oder eine Gruppe -SO- oder SO₂- enthält,
• R₄ ein Wasserstoffatom, ein Halogenatom, eine niedere Alkylgruppe mit 1 bis 6 Kohlenstoffatomen oder eine Gruppe -OR₆, wobei R₆ die unten angegebene Bedeutung besitzt,
wobei die oben angeführten Abkürzungen folgendes bedeuten:
- R₅ das gleiche wie R₄,
- R₆ ein Wasserstoffatom, eine geradkettige oder verzweigte Alkylgruppe mit 1 bis 20 Kohlenstoffatomen oder eine Gruppe -CO-R₁₁, wobei R₁₁ die unten angegebene Bedeutung besitzt,
- R₇ ein Wassserstoffatom oder eine niedere Alkylgruppe mit 1 bis 6 Kohlenstoffatomen,
- R₈:
(a) ein Wasserstoffatom,
(b) eine niedere Alkylgruppe mit 1 bis 6 Kohlenstoffatomen,
(c) eine Gruppe der Formel: 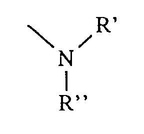 wobei R' und R'' die unten angegebene Bedeutung besitzen,
(d) eine Gruppe -OR₁₂, wobei R₁₂ die unten angegebene Bedeutung besitzt,
- R₉ und R₁₁, die gleich oder verschieden sein können, jeweils eine niedere Alkylgruppe mit 1 bis 6 Kohlenstoffatomen,
- R₁₀:
(i) eine Gruppe -OR₆,
(ii) eine Gruppe -CO-R₈,
(iii) eine Gruppe der Formel 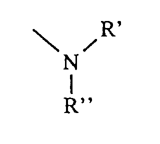 wobei R' und R'' die unten angegebene Bedeutung besitzen,
(iv) eine gegebenenfalls substituierte Arylgruppe,
(v) eine heterocyclische Gruppe,
(vi) eine cycloaliphatische C3- oder C6-Gruppe,
- R₁₂ ein Wasserstoffatom, eine geradkettige oder verzweigte Alkylgruppe mit 1 bis 20 Kohlenstoffatomen, eine Alkenylgruppe, eine mono- oder polyhydroxylierte Gruppe, eine gegebenenfalls substituierte Aryl- oder Aralkylgruppe, einen Zuckerrest oder einen Aminosäure- oder Peptidrest,
- R' und R'', die gleich oder verschieden sein können, ein Wasserstoffatom, eine niedere AIkylgruppe mit 1 bis 6 Kohlenstoffatomen, eine mono- oder polyhydroxylierte Gruppe, eine gegebenenfalls substituierte Arylgruppe oder einen Aminosäure-, Peptid- oder Zuckerrest oder zusammen einen Heterocyclus,
- m eine ganze Zahl von 1 bis 20,
- n eine ganze Zähl von 2 bis 20,
- t Null, 1 oder 2,
sowie die Salze und die optischen und geometrischen Isomeren dieser Verbindungen.

2. Verbindungen nach Anspruch 1, dadurch gekennzeichnet, daß sie in Form von Alkali- oder Erdalkalimetallsalzen, von Zinksalzen oder von Salzen eines organischen Amins vorliegen.

3. Verbindungen nach Anspruch 1 oder 2, dadurch gekennzeichnet, daß die niederen Alkylgruppen unter Methyl, Ethyl, Isopropyl, Butyl, tert-Butyl und Hexyl ausgewählt sind.

4. Verbindungen nach einem der vorhergehenden Ansprüche, dadurch gekennzeichnet, daß die geradkettigen oder verzweigten Alkylgruppen mit 1 bis 20 Kohlenstoffatomen unter Methyl, Ethyl, Propyl, 2-Ethylhexyl, Octyl, Dodecyl, Hexadecyl und Octadecyl ausgewählt sind.

5. Verbindungen nach einem der vorhergehenden Ansprüche, dadurch gekennzeichnet, daß die Monohydroxyalkylgruppen unter 2-Hydroxyethyl, 2-Hydroxypropyl und 3-Hydroxypropyl ausgewählt sind.

6. Verbindungen nach einem der vorhergehenden Ansprüche, dadurch gekennzeichnet, daß die Polyhydroxyalkylgruppen unter 2,3-Dihydroxypropyl, 2,3,4-Trihydroxybutyl, 2,3,4,5-Tetrahydroxypentyl sowie dem Pentaerythritylrest ausgewählt sind.

7. Verbindungen nach einem der vorhergehenden Ansprüche, dadurch gekennzeichnet, daß die Arylgruppe eine gegebenenfalls mit mindestens einem Halogenatom oder einer Hydroxyl- oder Nitrogruppe substituierte Phenylgruppe ist.

8. Verbindungen nach einem der vorhergehenden Ansprüche, dadurch gekennzeichnet, daß die Aralkylgruppen unter den gegebenenfalls mit mindestens einem Halogenatom oder einer Hydroxyl- oder Nitrogruppe substituierten Benzyl- und Phenylethylgruppen ausgewählt sind.

9. Verbindungen nach einem der vorhergehenden Ansprüche, dadurch gekennzeichnet, daß die Alkenylgruppen unter Gruppen mit 1 bis 5 Kohlenstoffatomen, die eine oder mehrere ethylenische Doppelbindungen aufweisen, insbesondere Allyl, ausgewählt sind.

10. Verbindungen nach einem der vorhergehenden Ansprüche, dadurch gekennzeichnet, daß die Zuckerreste unter Glucose-, Galactose-, Mannose- und Glucuronsäureresten ausgewählt sind.

11. Verbindungen nach einem der vorhergehenden Ansprüche, dadurch gekennzeichnet, daß die Aminosäurereste unter von Lysin, Glycin und Asparaginsäure abgeleiteten Gruppen ausgewählt sind.

12. Verbindungen nach einem der vorhergehenden Ansprüche, dadurch gekennzeichnet, daß die Peptidreste unter Dipeptid- und Tripeptidresten ausgewählt sind.

13. Verbindungen nach einem der vorhergehenden Ansprüche, dadurch gekennzeichnet, daß die heterocyclischen Gruppen ausgewählt sind unter Piperidino, Morpholino, Pyrrolidino und Piperazino, die gegebenenfalls in 4-Stellung mit einer C₁₋₆-Alkylgruppe oder einer Mono- oder Polyhydroxyalkylgruppe substituiert sind.

14. Verbindungen nach einem der vorhergehenden Ansprüche, dadurch gekennzeichnet, daß die Halogenatom unter Fluor, Chlor und Brom ausgewählt sind.

15. Verbindungen nach Anspruch 1, dadurch gekennzeichnet, daß sie unter folgenden Verbindungen ausgewählt sind:
- *anti*-4-[α-Hydroxyhexylimino-(5,6,7,8-tetrahydro-5,5,8,8-tetramethyl-2-naphthyl)acetamido]-benzoesäure
- syn-4-[α-Hydroxyhexylimino-(5,6,7,8-tetrahydro-5,5,8,8-tetramethyl-2-naphthyl)acetamido]-benzoesäure
- syn-4-[α-Methoxycarbonylpentylimino-(5,6,7,8-tetrahydro-5,5,8,8-tetramethyl-2-naphthyl)-acetamido]-benzoesäure
- syn-4-[α-Hydroxyoctyloxyimino-(5,6,7,8-tetrahydro-5,5,8,8-tetramethyl-2-naphthyl)-acetamido]-benzoesäure
- trans-4-[α-Hydroxyoctyloxyimino-(5,6,7,8-tetrahydro-5,5,8,8-tetramethyl-2-naphthyl)-acetamido]-benzoesäure
- syn-4-[α-Hydroxyheptyloxyimino-(5,6,7,8-tetrahydro-5,5,8,8-tetramethyl-2-naphthyl)-acetamido]-benzoesäure
- trans-4-[α-Hydroxyheptyloxyimino-(5,6,7,8-tetrahydro-5,5,8,8-tetramethyl-2-naphthyl)-acetamido]-benzoesäure
- syn-4-[α-Hydroxypropyloxyimino-(5,6,7,8-tetrahydro-5,5,8,8-tetramethyl-2-naphthyl)-acetamido]-benzoesäure
- trans-4-[α-Hydroxypropyloxyimino-(5,6,7,8-tetrahydro-5,5,8,8-tetramethyl-2 naphthyl)-acetamido]-benzoesäure
- syn-4-[α-Ethoxycarbonylpropyloxyimino-(5,6,7,8-tetrahydro-5,5,8,8-tetramethyl-2-naphthyl)-acetamido]-benzoesäure
- syn-4-[α-Hydroxydecyloxyimino-(5,6,7,8-tetrahydro-5,5,8,8-tetramethyl-2-naphthyl)-acetamido]-benzoesäure
- syn-4-[α-Hydroxynonyloxyimino-(5,6,7,8-tetrahydro-5,5,8,8-tetramethyl-2-naphthyl)-acetamido]-benzoesäure
- syn-4-[α-Benzyloxyimino-(5,6,7,8-tetrahydro-5,5,8,8-tetramethyl-2-naphthyl)-acetamido]-benzoesäure
- syn-4-[α-Methoxyethoxymethoxyimino-(5,6,7,8-tetrahydro-5,5,8,8-tetramethyl-2-naphthyl)-acetamido]-benzoesäure
- syn-4-[α-Ethoxycarbonylpropyloxyimino-(5,6,7,8-tetrahydro-5,5,8,8-tetramethyl-2-naphthyl)-acetamido]-allylbenzoat
- (E)-4-[2-(5,6,7,8-Tetrahydro-5,5,8,8-tetramethyl-2-naphthyl)-2-nonenamido]benzoesäure
- (Z)-4-[2-(5,6,7,8-Tetrahydro-5,5,8,8-tetramethyl-2-naphthyl)-2-nonenamido]benzoesäure
- (E)-4-[2-(5,6,7,8-Tetrahydro-5,5,8,8-tetramethyl-2-naphthyl)-2-undecenamido]benzoesäure
- (E)-4-[2-(5,6,7,8-Tetrahydro-5,5,8,8-tetramethyl-2-naphthyl)-2-undecenamido]phenylcarboxamid
- (E)-4-[2-(5,6,7,8-Tetrahydro-5,5,8,8-tetramethyl-2-naphthyl)-2-undecenamido]benzylalkohol
- (E)-4-[2-(5,6,7,8-Tetrahydro-5,5,8,8-tetramethyl-2-naphthyl)-2-undecenamido]phenol
- (Z)-4-[2-(5,6,7,8-Tetrahydro-5,5,8,8-tetramethyl-2-naphthyl)-2-undecenamido]benzoesäure
- (E)-4-[2-(5,6,7,8-Tetrahydro-5,5,8,8-tetramethyl-2-naphthyl)-2-hexadecenamido]benzoesäure
und
- (Z)-4-[2-(5,6,7,8-Tetrahydro-5,5,8,8-tetramethyl-2-naphthyl)-2-hexadecenamido]benzoesäure.

16. Verbindungen nach Anspruch 1, dadurch gekennzeichnet daß die Verbindungen der Formel (I) Verbindungen sind, bei denen R₁ die Gruppe -COR₈ darstellt und X und Y gemeinsam eine Gruppe bilden, die ausgewählt ist unter den Gruppen der folgenden Formeln:
-
=N-O-(CH₂)ₙ-R₁₀,
-
=CH-(CH₂)ₘ-R₁₀,
-
=N-O-(CH₂)₂-O-CH₂-CH₃,
-
=N-O-CH₂-O-CH₂-CH₂-O-CH₃,
-
=CH-(CH₂)ₘ-CH₃,
wobei R₈, R₁₀, m und n die in Anspruch 1 angegebene Bedeutung haben.

17. Verbindungen nach einem der vorhergehenden Ansprüche zur Verwendung als Arzneimittel.

18. Verbindungen nach Anspruch 17 zur Verwendung als Arzneimittel zur Behandlung von dermatologischen und rheumatischen Erkrankungen sowie von Atemwegs-, Herz- und Gefäß- und Augenerkrankungen.

19. Verwendung mindestens einer Verbindung nach den Ansprüchen 1 bis 16 zur Herstellung von Arzneimitteln zur Behandlung von dermatologischen, rheumatischen, Atemwegs-, Herz- und Gefäß- und Augenerkrankungen.

20. Pharmazeutische Zusammensetzung, dadurch gekennzeichnet, daß sie in einem pharmazeutisch akzeptablen Träger mindestens eine Verbindung nach einem der Ansprüche 1 bis 16 enthält.

21. Zusammensetzung nach Anspruch 20, dadurch gekennzeichnet, daß die Konzentration der Verbindung(en) nach einem der Ansprüche 1 bis 16 im Bereich von 0,001 bis 5 Gew.-%, bezogen auf die gesamte Zusammensetzung, beträgt.

22. Kosmetische Zusammensetzung, dadurch gekennzeichnet, daß sie in einem kosmetisch akzeptablen Träger mindestens eine Verbindung nach einem der Ansprüche 1 bis 16 enthält.

23. Zusammensetzung nach Anspruch 22, dadurch gekennzeichnet, daß die Konzentration der Verbindung(en) nach einem der Ansprüche 1 bis 16 im Bereich von 0,001 bis 3 Gew.-%, bezogen auf die gesamte Zusammensetzung, beträgt.

24. Verwendung einer kosmetischen Zusammensetzung nach Anspruch 22 oder 23 für die Körper- oder Haarpflege.
